# EUROPÄISCHE PATENTSCHRIFT

(11) **EP 0 664 297 B1**
(45) Veröffentlichungstag und Bekanntmachung des Hinweises auf die Patenterteilung: **08.04.1998**
(21) Anmeldenummer: 95100198.1
(22) Anmeldetag: 09.01.1995
(51) Int. Cl.: C07K 11/02, C07D 273/00, A61K 31/365

(54) **Verwendung von cyclischen Depsipeptiden mit 12 Ringatomen zur Bekämpfung von Endoparasiten, neue cyclische Depsipeptide mit 12 Ringatomen und Verfahren zu ihrer Herstellung**
Use of cyclic Didepsipeptides having 12 ring atoms to control endoparasites, novel cyclic depsipeptides with 12 ring atoms and process for their synthesis
Utilisation des depsipeptides cycliques avec 12 atomes cycliques dans la lutte contre des endoparasites, nouveaux depsipeptides cycliques avec 12 atomes cycliques et procédé de leur préparation

(30) Priorität: 19.01.1994 DE 4401389
(43) Veröffentlichungstag der Anmeldung: 26.07.1995
(73) Patentinhaber: BAYER AG, 51368 Leverkusen (DE)
(72) Erfinder: Scherkenbeck, Jürgen, Dr., D-42929 Wermelskirchen (DE); Jeschke, Peter, Dr., D-51373 Leverkusen (DE); Plant, Andrew, Dr., D-51373 Leverkusen (DE); Harder, Achim, Dr., D-51109 Köln (DE); Mencke, Norbert, Dr., D-51381 Leverkusen (DE)

(56) Entgegenhaltungen:
- EP-A- 0 382 173
- WO-A-93/25543
- KEMIAI KOEZLEMENYEK, Bd. 60, Seite 10 VAINSTEIN B.K. ET AL. 'Enniantin és Valinomicin sorba tartozo Antibiotikumok vizsgalata: Az Enniantin B segitségével történö ion szallitas szerkezeti szempontjai'
- J.AM.CHEM.SOC.USA, Bd. 91, Seite 4888 J.KONNERT ET AL. 'The Conformation and Crystal Structure of the Cyclotetradepsipeptide D-HyIv-L-MeILeu-D-HyIv-L-MeLeu'

## Beschreibung

Die vorliegende Erfindung betrifft die Verwendung von cyclischen Depsipeptiden mit 12 Ringatomen zur Bekämpfung von Endoparasiten, neue cyclische Depsipeptide mit 12 Ringatomen und Verfahren zu ihrer Herstellung.

Bestimmte cyclische Depsipeptide mit 12 Ringatomen und Verfahren zu ihrer Herstellung sind bereits bekannt (vgl. z.B.: J. Am. Chem. Soc. 91 (1969), S. 4888; Biorg. Khim. 1 (1975), 375; Biofizika 16 (1971) S. 407; Iav. Akad. Nark. SCSR, Set Khim, 1970, S. 991).

Über eine Verwendung dieser Verbindungen gegen Endoparasiten ist jedoch bisher nichts bekannt geworden. Aus EP-A 382 173 und WO 93/25 543 sind endoparasitizide cyclische Depsipeptide mit 24 bzw. 18 Ringatomen bekannt geworden.

Die vorliegende Erfindung betrifft:
1. Die Verwendung von cyclischen Depsipeptiden mit 12 Ringatomen der allgemeinen Formel ( I ) 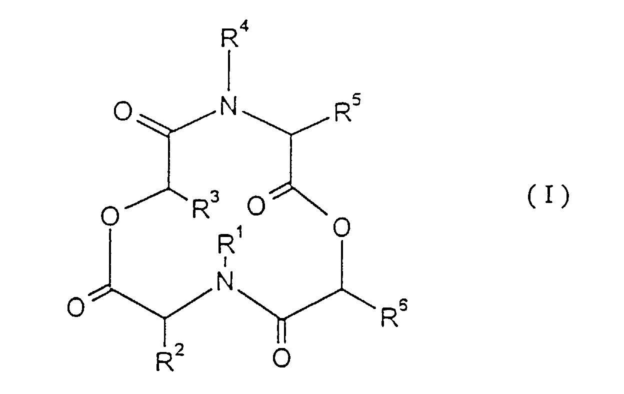 in welcher die Reste die folgende Bedeutung haben
   - R¹ und R⁴: stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-Alkyl, Phenyl-C₁-C₄-alkyloxy-C₁-C₆-alkyl, Mercapto-C₁-C₆-alkyl, C₁-C₄-Alkylthio-C₁-C₆-alkyl, C₁-C₄-Alkylsulfinyl-C₁-C₆-alkyl, C₁-C₄-Alkylsulfonyl-C₁-C₆-alkyl, Carboxy-C₁-C₆-alykl C₁-C₄-Alkoxycarbonyl-C₁-C₆-alkyl, C₁-C₄-Phenylalkoxycarbonyl-C₁-C₆, Carbamoyl-C₁-C₆-alkyl, Amino-C₁-C₆-alkyl, C₁-C₄-Alkylamino-C₁-C₆-alkyl,C₁-C₄-Dialkylamino C₁-C₆-alkyl, Guanido-C₁-C₆-alkyl, C₁-C₄-Alkoxycarbonylamino-C₁-C₆-alkyl, 9-Fluorenylmethoxycarbonyl(Fmoc)amino-C₁-C₆-alkyl, C₃-C₇-Cycloalkyl, C₃-C₇-Cycloalkyl-C₁-C₄-alkyl, Phenyl, Phenyl-C₁-C₄-alkyl, die gegebenenfalls durch Reste aus der Reihe Halogen, Hydroxy, Nitro, CN, C₁-C₄-Alkoxy, C₁-C₄-Alkyl, substituiert sein können;
   - R²_{,} R³, R⁵ und R⁶: stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-alkyl, Phenyl-C₁-C₄-alkyloxy-C₁-C₆-alkyl, Mercapto-C₁-C₆-alkyl, C₁-C₄-Alkylthio-C₁-C₆-alkyl, C₁-C₄-Alkylthio-C₁-C₆-alkyl, C₁-C₄-Alkylsulfonyl-C₁-C₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Phenylalkoxycarbonyl-C₁-C₆-alkyl, Carbamoyl-C₁-C₆-alkyl, Amino-C₁-C₆-alkyl, C₁-C₄-Alkylamino-C₁-C₆-alkyl, alkyl, Amino-C₁-C₆-alkyl, C₁-C₄-Alkylamino-C₁-C₆-alkyl, C₁-C₄-Dialkylamino-C₁-C₆-alkyl, C₂-C₈-Alkenyl, C₃-C₇-Cycloalkyl, C₃-C₇-Cycloalkyl-C₁-C₄-alkyl, Phenyl-C₁-C₄-alkyl, Thienylmethyl, Thiazolylmethyl, Pyridylmethyl, die gegebenenfalls durch Reste aus der Reihe Halogen, Hydroxy, Sulfonyl (SO₃H), CN, NO₂, Amino, Di(C₁-C₄-alkyl)amino, C₁-C₄-Alkoxy, C₁-C₄-Alkyl substituiert sein können,
   sowie deren optische Isomere und Racemate
   zur Herstellung von Mitteln zur Bekämpfung von Endoparasiten in der Medizin und Tiermedizin.
2. Neue Cyclische Depsipeptide mit 12 Ringatomen der Formel (I) 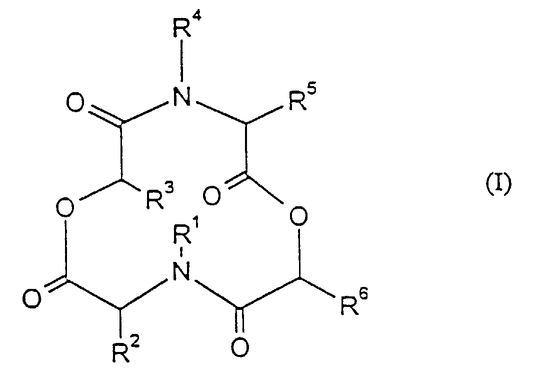 in welcher die Reste die folgende Bedeutung haben
   - R¹ und R⁴: stehen unabhängig voneinander für Wasserstoff, geradkettiges oder verzweigtes C₁-C₈-Alkyl, 1-5 Halogen-C₁₋₄-alkyl, C₃-C₆-Cycloalkyl, Phenyl, Phenyl-C₁-C₄-alkyl, die gegebenenfalls durch Reste aus der Reihe Halogen, Hydroxy, Nitro, CN, C₁-C₄-Alkoxy, C₁-C₄-Alkyl, substituiert sein können,
   - R², R³, R⁵: stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-alkyl, Mercapto-C₁-C₆-alkyl, C₁-C₄-Alkylthio-C₁-C₆-alkyl, C₁-C₄-Alkylsulfinyl-C₁₋₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Phenylalkoxycarbonyl-C₁-C₆ -alkyl, Carbamoyl-C₁-C₆-alkyl, Amino-C₁-C₆-alkyl, C₁-C₄-Alkylamino-C₁-C₆-alkyl, C₁-C₄-Dialkylamino-C₁-C₆-alkyl, C₂-C₈-Alkenyl, C₃-C₇-Cycloalkyl, C₃-C₇-Cycloalkyl-C₁-C₄-alkyl, Phenyl, Phenyl-C₁-C₄-alkyl, Thienylmethyl, Thiazolylmethyl, Pyridylmethyl, die gegebenenfalls durch Reste aus der Reihe Halogen, Hydroxy, Sulfonyl (SO₃H), CN, NO₂, Amino, Di (C₁-C₄-alkyl)amino, C₁-C₄-Alkyl, substituiert sein können;
   - R⁶ steht für: 1-5 Halogen-C₁₋₄-alkyl, Hydroxy-C₁-C₆-alkyl, C₁-C₄-Alkanoyloxy-C₁-C₆-alkyl, C₁-C₄-alkoxy-C₁-C₆-alkyl, Mercapto-C₁-C₆-alkyl, C₁-C₄-Alkylthio-C₁-C₆-alkyl, C₁-C₄-Alkylsulfinyl-C₁₋₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Phenylalkoxycarbonyl-C₁*C₆ -alkyl, Carbamoyl-C₁-C₆-alkyl, Almino-C₁-C₆-alkyl, C₁-C₄-Alkylamino-C₁-C₆-alkyl, C₁-C₄-Dialkylamino-C₁-C₆-alkyl, C₂-C₈-Alkenyl, C₃-C₇-Cycloalkyl, C₃-C₇-Cycloalkyl-C₁-C₄-alkyl, Phenyl, Phenyl-C₁-C₄-alkyl, Thienylmethyl, Thiazolylmethyl, Pyridylmethyl, die gegebenenfalls durch Reste aus der Reihe Halogen, Hydroxy, Sulfonyl (SO₃H), CN, NO₂, Amino, Di (C₁-C₄-alkyl)amino, C₁-C₄-Alkyl, substituiert sein können;
   sowie deren optische Isomere und Racemate
   mit der Maßgabe für den Fall, daß einer der Reste R¹ und R⁴ für einen anderen als einen Methylrest steht, der Rest R⁶ zusätzlich zu den angegebenen Bedeutungen für Wasserstoff oder geradkettiges oder verzweigtes C₁₋₉-Alkyl stehen kann.
3. Verfahren zur Herstellung der neuen cyclischen Depsipeptide mit 12 Ringatomen der Formel (I) gemäß Punkt 2 (oben). 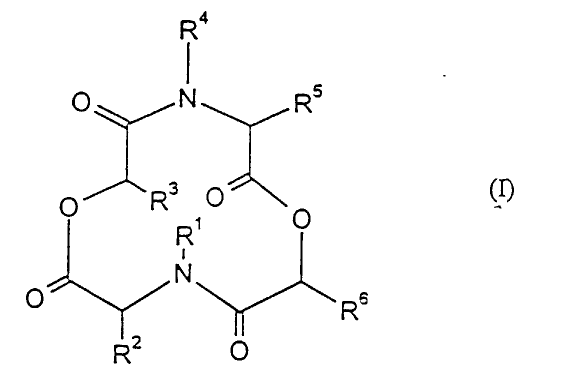 in welcher
   die Reste R¹-R⁶ die unter Punkt 2 angegebene Bedeutung haben,
   dadurch gekennzeichnet, daß man offenkettige Tetradepsipeptide der Formel (II) 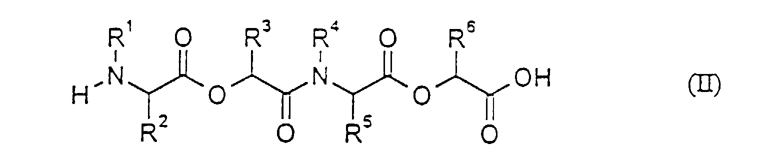 in welcher
   - R¹ bis R⁶: die oben angegebene Bedeutung haben,
   in Gegenwart eines Verdünnungsmittels und in Gegenwart eines Kopplungsreagenzes cyclisiert.
   - R¹ bis R⁶: die oben angegebene Bedeutung haben,
   in Gegenwart eines Verdünnungsmittel und in Gegenwart eines Kopplungsreagenzes cyclisiert.

Offenkettige Tetradepsipeptide der Formel (II) 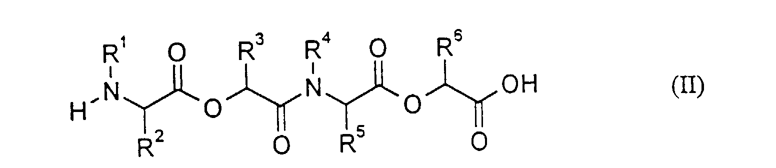 in welcher
- R¹ bis R⁶: die oben angegebene Bedeutung haben. werden hergestellt, indem man
1a) Verbindungen der Formel (III) 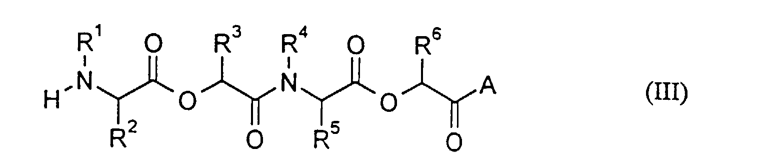 in welcher
   A für tert.-Butoxy steht,
   R¹ bis R⁶ die oben angegebene Bedeutung besitzen, in Gegenwart eines Verdünnungsmittels und einer Protonensäure verseift,
   oder
1b) Verbindungen der Formel (IV) 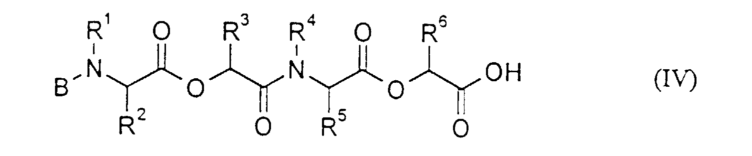 in welcher
   B für Benzyl steht,
   R¹ bis R⁶ die oben angegebene Bedeutung besitzen, in Gegenwart eines Verdünnungsmittels und eines Katalysators hydrogenolysiert.

Verbindungen der Formel (III) 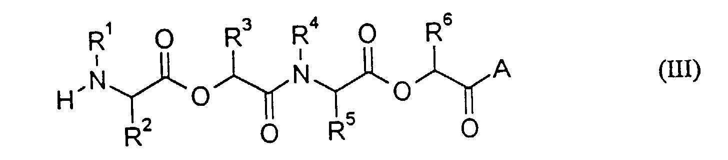 in welcher
- A: für tert.-Butoxy und
- R¹ bis R⁶: die oben angegebene Bedeutung besitzen.
werden hergestellt, indem man Verbindungen der Formel (V) 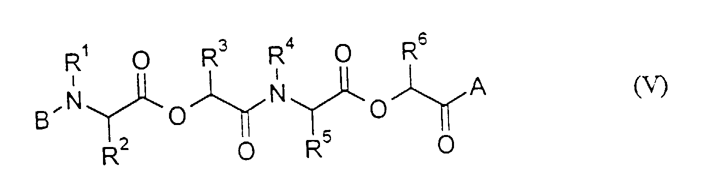 in welcher
- A: für tert.-Butoxy,
- B: für Benzyl und
- R¹ bis R⁶: die oben angegebene Bedeutung besitzen,
in Gegenwart eines Verdünnungsmittels und eines Katalysators hydrogenolisiert.

Verbindungen der Formel (IV) 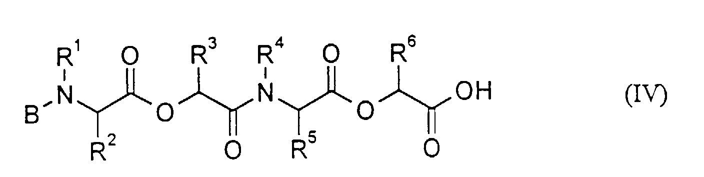 in welcher
- B: für Benzyl steht und
- R¹ bis R⁶: die oben angegebene Bedeutung besitzen.
werden hergestellt, indem man Verbindungen der Formel (V) 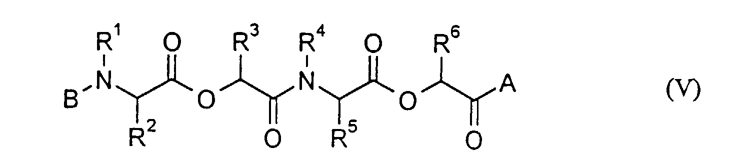 in welcher
- B: für Benzyl,
- A: für tert.-Butoxy und
- R¹ bis R⁶: die oben angegebene Bedeutung besitzen,
in Gegenwart eines Verdünnungsmittels und einer Protonensäure verseift.

Verbindungen der Formel (V) 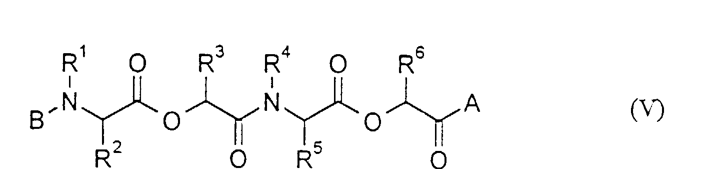 in welcher
- B: für Benzyl,
- A: für tert.-Butoxy und
- R¹ bis R⁶: die oben angegebene Bedeutung haben.
werden hergestellt, indem man Didepsipeptide der Formel (VI) 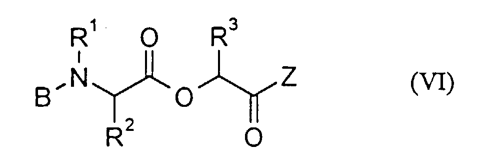 in welcher
- B: für Benzyl,
- Z: für OH oder Cl steht,
- R¹ bis R³: die oben angegebene Bedeutung haben,
und Didepsipeptide der Formel (VII) 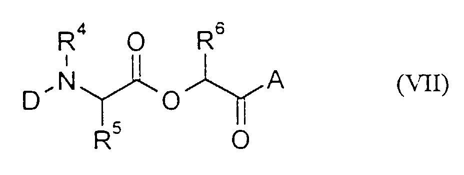 in welcher
- D: für Wasserstoff und
- A: für tert.-Butoxy steht, sowie
- R⁴ bis R⁶: die oben angegebene Bedeutung haben,
in Gegenwart eines Verdünnungsmittels und eines geeigneten Kopplungsreagenzes kondensiert.

Didepsipeptide der Formel (VI) 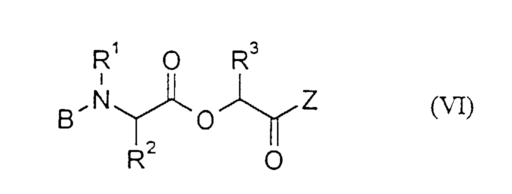 in welcher
- B: für Benzyl,
- Z: für OH oder Cl steht sowie
- R¹ bis R³: die oben angegebene Bedeutung besitzen.
werden hergestellt, indem man eine Verbindung der Formel (VIII) 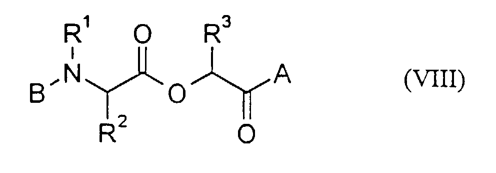 in welcher
- A: für tert.-Butoxy und
- B: für Benzyl steht, sowie
- R¹ bis R³: die oben angegebene Bedeutung besitzen,
in Gegenwart eines Verdünnungsmittels und einer Protonensäure verseift.

Didepsipeptide der Formel (VII) 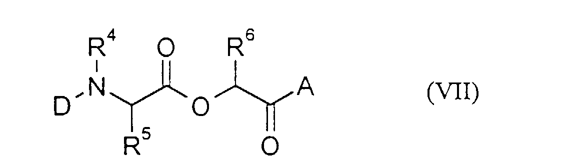 in welcher
- D: für Wasserstoff und
- A: für tert.-Butoxy steht, sowie
- R⁴ bis R⁶: die oben angegebene Bedeutung besitzen
werden hergestellt,indem man Verbindungen der Formel (IX) 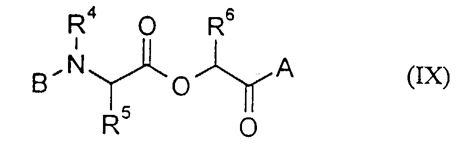 in welcher
- A: für tert.-Butoxy,
- B: für Benzyl steht, sowie
- R⁴ bis R⁶: die oben angegebene Bedeutung haben,
in Gegenwart eines Verdünnungsmittels und eines Katalysators hydrogenolysiert.

Didepsipeptide der Formel (VIII) 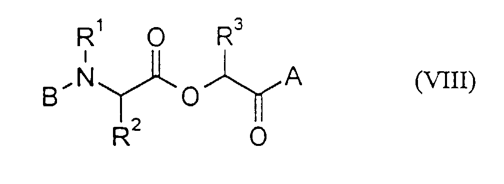 in welcher
- A: für tert.-Butoxy und
- B: für Benzyl steht, sowie
- R¹ bis R³: die oben angegebene Bedeutung besitzen.
werden hergestellt, indem man eine Aminocarbonsäure der Formel (X) 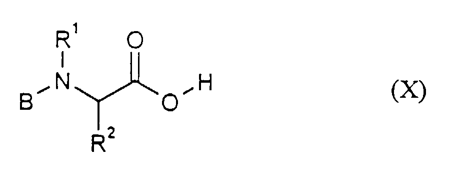 in welcher
- B: für Benzyl steht und
- R¹ und R²: die oben angegebene Bedeutung besitzen,
in Form ihres Alkalimetallsalzes, bevorzugt ihres Caesiumsalzes,
und eine α-Halogencarbonsäure der Formel (XI) 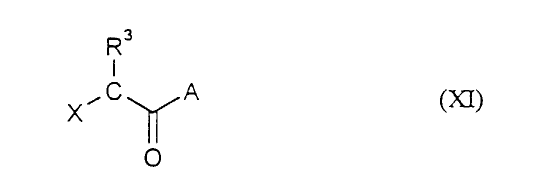 in welcher
- X: für Cl oder Br und
- A: für tert.-Butoxy steht sowie
- R³: die oben angegebene Bedeutung besitzt,
in Gegenwart eines Verdünnungsmittels koppelt

Didepsipeptide der Formel (IX) 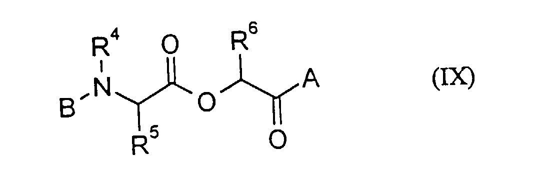 in welcher
- A: für tert.-Butoxy steht und
- B: für Benzyl sowie
- R⁴ bis R⁶: die oben angegebene Bedeutung besitzen.
werden hergestellt, indem man eine Aminocarbonsäure der Formel (XII) 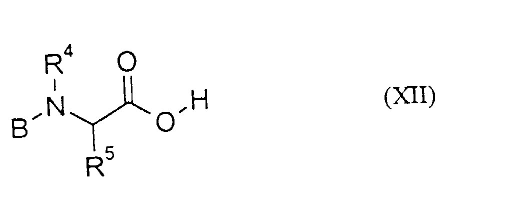 in welcher
- B: für Benzyl steht und
- R⁴ und R²: die oben angegebene Bedeutung besitzen,
in Form ihres Alkalimetallsalzes, bevorzugt ihres Caesiumsalzes, mit einer α-Halogencarbonsäure der Formel (XIII) 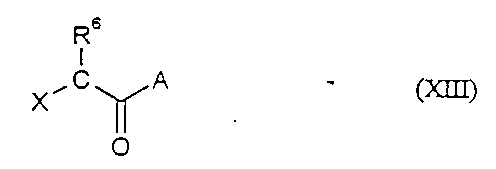 in welcher
- X: für Cl oder Br und
- A: für tert.-Butoxy steht sowie
- R⁶: die oben angegebene Bedeutung besitzt,
in Gegenwart eines Verdünnungsmittels koppelt.

Die cyclischen Depsipeptide mit 12 Ringatomen der Formel (I) sowie deren Säureadditions-Salze und Metallsalz-Komplexe besitzen sehr gute endoparasitizide, insbesondere anthelmintische Wirkung und können bevorzugt im Bereich der Veterinärmedizin eingesetzt werden. Überraschenderweise zeigen die erfindungsgemäßen Stoffe bei der Bekämpfung von Wurmerkrankungen eine deutlich bessere Wirksamkeit als vorbekannte Verbindungen gleicher Wirkungsrichtung.

Gegebenenfalls substituiertes Alkyl allein oder als Bestandteil eines Restes in den allgemeinen Formeln bedeutet geradkettiges oder verzweigtes Alkyl mit vorzugsweise 1 bis 8, insbesondere 1 bis 5 Kohlenstoffatomen. Beispielhaft und vorzugsweise seien gegebenenfalls substituiertes Methyl, Ethyl, n- und i-Propyl, n-, i- und t-Butyl, genannt.

Gegebenenfalls substituiertes Alkenyl allein oder als Bestandteil eines Restes in den allgemeinen Formeln bedeutet geradkettiges oder verzweigtes Alkenyl mit 2 bis 8 Kohlenstoffatomen. Beispielhaft und vorzugsweise seien gegebenenfalls substituiertes Ethenyl, Propenyl-(1), Propenyl-(2) und Butenyl-(3) genannt.

Gegebenenfalls substituiertes Cycloalkyl in den allgemeinen Formeln bedeutet mono-, bi- und tricyclisches Cycloalkyl mit vorzugsweise 3 bis 7 , insbesondere 3, 5 oder 6 Kohlenstoffatomen. Beispielsweise und vorzugsweise seien gegebenenfalls substituiertes Cyclopropyl, Cyclobutyl, Cyclopentyl, Cyclohexyl, Cycloheptyl, Bicyclo[2.2.1]heptyl, Bicyclo[2.2.2]octyl und Adamantyl genannt.

Gegebenenfalls substituiertes Alkoxy in den allgemeinen Formeln bedeutet geradkettiges oder verzweigtes Alkoxy mit 1 bis 4 Kohlenstoffatomen. Beispielhaft und vorzugsweise seien gegebenenfalls substituiertes Methoxy, Ethoxy, n- und i-Propoxy und n-, o- und t-Butoxy genannt.

Gegebenenfalls substituiertes Alkylthio in den allgemeinen Formeln bedeutet geradkettiges oder verzweigtes Alkylthio mit vorzugsweise 1 bis 6, insbesondere 1 bis 4 Kohlenstoffatomen. Beispielhaft und vorzugsweise seien gegebenenfalls substituiertes Methylthio, Ethylthio, n- und i-Propylthio, n-, o- und t-Butylthio genannt.

Halogenalkyl in den allgemeinen Formeln enthält 1 bis 4, insbesondere 1 oder 2 Kohlenstoffatome und vorzugsweise 1 bis 9, insbesondere 1 bis 5 gleiche oder verschiedene Halogentome, wobei als Halogenatome vorzugsweise Fluor, Chlor und Brom, insbesondere Fluor und Chlor stehen. Beispielhaft seien Trifluormethyl, Chlor-di-fluormethyl, Brommethyl, 2,2,2-Trifluorethyl und Pentafluorethyl, Perfluor-t-butyl genannt.

Als gegebenenfalls substituiertes Arylalkyl in den allgemeinen Formeln sei beispielhaft und vorzugsweise gegebenenfalls substituiertes Benzyl und Phenylethyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt.

Alkyl mit vorzugsweise 1 bis 4, insbesondere 1 oder 2 Kohlenstoffatomen, wie Methyl, Ethyl, n- und i-Propyl und n-, i- und t-Butyl: Alkoxy mit vorzugsweise 1 bis 4, insbesondere 1 oder 2 Kohlenstoffatomen, wie Methoxy, Ethoxy, n- und i- Propyloxy und n-, i- und t-Butyloxy; Hydroxy; Halogen, vorzugsweise Fluor, Chlor, Brom und Iod, insbesondere Fluor, Chlor und Brom; Cyano, Nitro; Amino;

Bevorzugt sind Verbindungen der Formel ( I ),
in welcher
- R¹ und R⁴: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-alkyl, insbesondere Hydroxymethyl, 1-Hydroxyethyl, C₁-C₄-Alkannyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyt 1-Benzyloxyethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl(Fmoc)amino-C₁-C₆-alkyl, insbesondere 9-Fluorenyl-methoxycarbonyl(Fmoc)aminopropyl, 9-Fluorenylmethoxycarbonyl(Fmoc)aminobutyl, C₃-C₇-Cycloalkyl, insbesondere Cyclopentyl, Cyclohexyl, Cycloheptyl, C₃-C₄-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl, die gegebenenfalls durch Reste aus der Reihe Halogen, insbesondere Fluor, Chlor Brom oder Iod, Hydroxy, Nitro, CN, C₁-C₄- Alkoxy, insbesondere Methoxy oder Ethoxy, C₁-C₄-Alkyl, insbesondere Methyl, substituiert sein können,
- R², R³, R⁵ und R⁶: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-alkyl, insbesondere Hydroxymethyl, 1-Hydroxyethyl,C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl,C₁-C₄-Alkylsulfonyl-C₁-C₆-alkyl, insbesondere Methylsulfonylethyl, Carboxy-C₁ -C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl, Thienylmethyl, Thiazolylmethyl, Pyridylmethyl, die gegebenenfalls durch Reste aus der Reihe Halogen, insbesondere Fluor, Chlor, Brom oder Iod, Hydroxy, Sulfonyl (SO₃H), CN, NO₂, Amino, Di(C₁-C₄-alkyl)amino z.B. Dimethylamino, C₁-C₄-Alkoxy, insbesondere Methoxy oder Ethoxy, C₁-C₄-Alkyl, insbesondere Methyl, substituiert sein können,
sowie deren optische Isomere und Racemate

Besonders bevorzugt sind Verbindungen der Formel ( I ),
in welcher
- R¹ und R⁴: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.- Hexyl, Heptyl, Isoheptyl, sec.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl, die gegebenenfalls durch einen oder mehrere gleiche oder verschiedene der oben angegebenen Reste substituiert sein können,
- R², R³, R⁵ und R⁶: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, Hydroxy-C₁-C₆-blkyl, insbesondere Hydroxymethyl, Aryl-C₁-C₄-alkyloxy-C₁-C₆-alkyl, insbesondere Benzyloxymethyl, 1-Benzyloxyethyl, Carboxy-C₁-C₆-blkyl, insbesondere Carboxymethyl, Carboxyethyl, C₁-C4-Alkoxycarbonyl-C₁-C₆-alkyl, insbesondere Methoxycarbonylmethyl, Ethoxycarbonylethyl, C₁-C₄-Aryl-alkoxycarbonyl-C₁-C₆-alkyl, insbesondere Benzyloxycarbonylmethyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₃-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptyl-methyl, Phenyl, Phenyl-C₁-C₄-alkyl, insbesondere Phenylmethyl, Thienylmethyl, die gegebenenfalls durch einen oder mehrere gleiche oder verschiedene der oben angegebenen Reste substituiert sein können,
sowie deren optische Isomere und Racemate.

Ganz besonders bevorzugt sind Verbindungen der Formel ( I ),
in welcher
- R¹ und R⁴: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.- Hexyl, Heptyl, Isoheptyl, sec.-Heptyl, Oktyl, Isooktyl, sec.-Oktyl, C₃-C₇Cycloalkyl-C₁-C₄-alkyl, insbesondere Cyclohexylmethyl, Phenyl-C₁-C₄-alkyl, insbesondere Phenylmethyl stehen,
- R², R³, R⁵ und R⁶: unabhängig voneinander für Wasserstoff, geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, 1-5 Halogen-C₁₋₄-alkyl, insbesondere Trichlormethyl, Trifluormethyl, Pentafluorethyl, Chlorfluorethyl, C₂-C₈- Alkenyl, insbesondere Vinyl, Allyl, C₃-C₇-Cycloalkyl-C₁-C₄-alkyl, insbesondere Cyclohexylmethyl, Phenyl-C₁-C₄-alkyl, insbesondere Phenylmethyl, Thienylmethyl, die gegebenenfalls durch einen oder mehrere gleiche oder verschiedene der oben angegebenen Reste substituiert sein können,
sowie deren optische Isomere und Racemate.

Die Verbindungen der allgemeinen Formel (I), können in optisch aktiven, stereoisomeren Formen oder als racemische Gemische vorliegen und verwendet werden. Vorzugsweise werden die optisch aktiven, stereoisomeren Formen der Verbindungen der allgemeinen Formel (I) verwendet.

Im einzelnen seien folgende Verbindungen der allgemeinen Formel ( I ) genannt, in welcher
die Reste R¹ bis R⁶ die folgende Bedeutung haben:

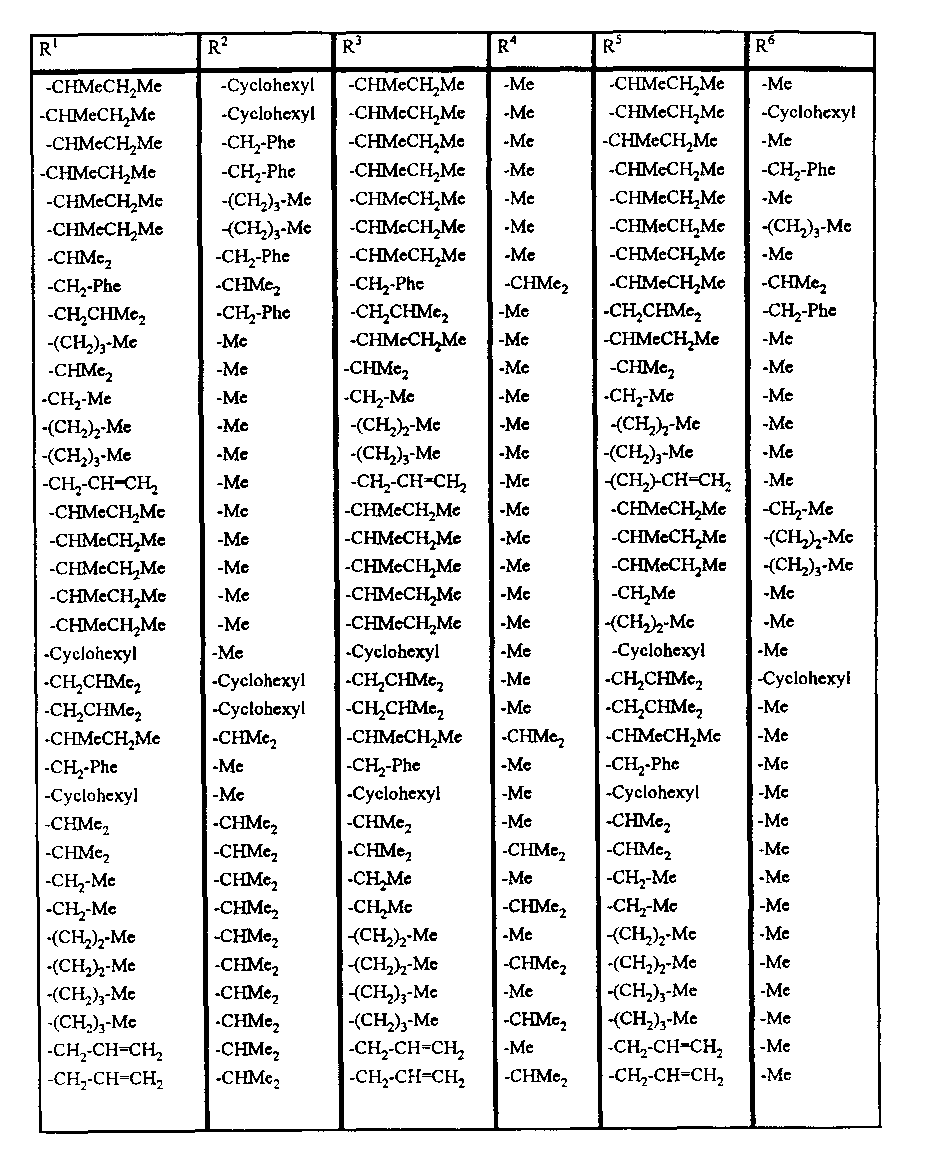

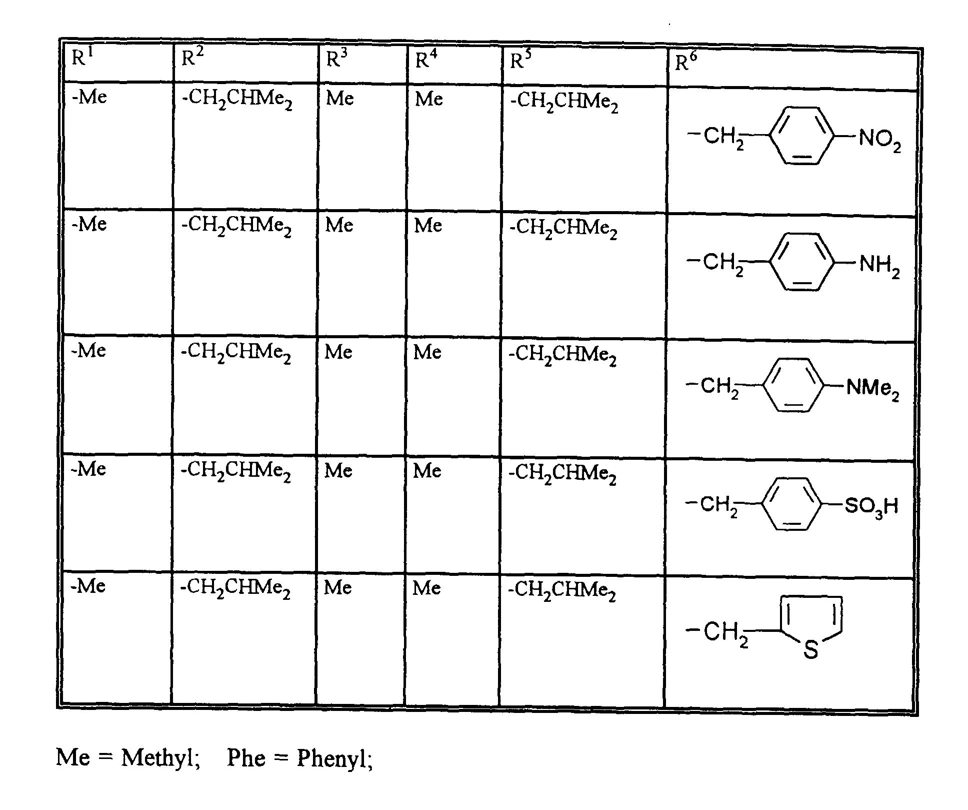

Von den neuen Verbindungen der allgemeinen Formel (Ia) sind diejenigen bevorzugt und besonders bevorzugt in denen die Substituenten die oben angegebenen bevorzugten Definitionen besitzen.

Die Verbindungen der allgemeinen Formel (I) sind teilweise bekannt (s. oben) oder können nach den dort angegebenen Verfahren erhalten werden.

Die neuen Verbindungen der Formel (I) lassen sich nach dem von U. Schmidt et al. für makrocyclische Peptidalkaloide angewendeten Verfahren (vgl. z.B.: U. Schmidt et al. in Synthesis (1991) S. 294-300 [Didemnin A, B und C]; Angew. Chem. 96 (1984) S. 723-724 [Dolastatin 3]; Angew. Chem. 102 (1990) S. 562-563 [Fenestin A]; Angew. Chem. 97 (1985) S. 606-607 [Ulicyclamid]; J. Org. Chem. 47 (1982) S. 3261-3264) herstellen.

Die Verbindungen der allgemeinen Formel (I) lassen sich nach den oben unter Punkt 3 angegebenen Verfahren herstellen.

Setzt man bei Verfahren 3 zur Herstellung der neuen cyclischen Depsipeptide (I) als Verbindungen der Formel (II) N-Methyl-L-isoleucyl-D-lactyl-N-methyl-L-isoleucyl-D-milchsäure ein, so läßt sich das Verfahren durch folgendes Reaktionsschema wiedergeben:

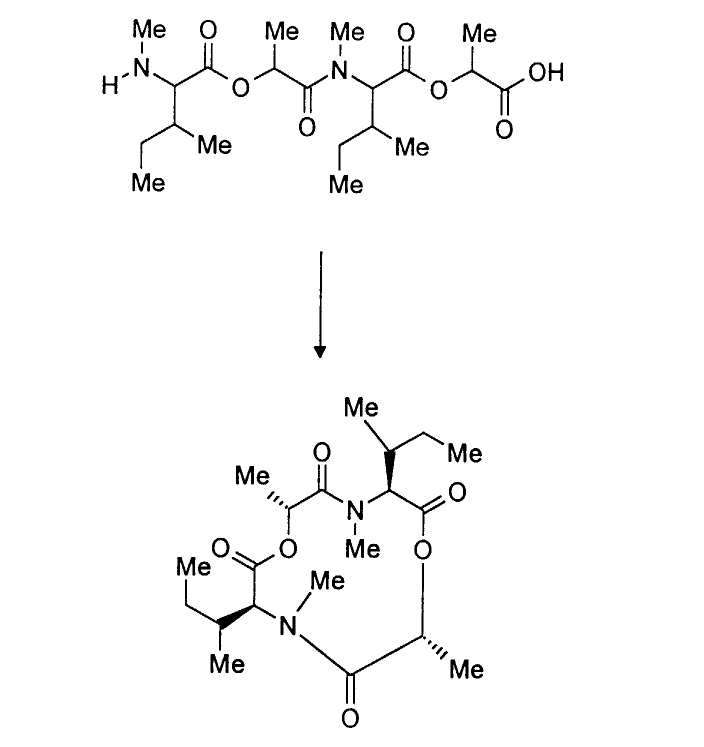

Die zur Durchführung des Verfahrens 3 als Ausgangsstoffe benötigten offenkettigen Tetradepsipeptide sind durch die Formel (II) allgemein definiert. In dieser Formel stehen R¹ bis R⁶ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten Tetradepsipeptide der Formel (II) sind neu. Ihre Herstellung wird weiter unten beschrieben.

Im einzelnen seien folgende Verbindungen der allgemeinen Formel (II) genannt, in welcher die Reste R¹ bis R⁶ die folgende Bedeutung haben:

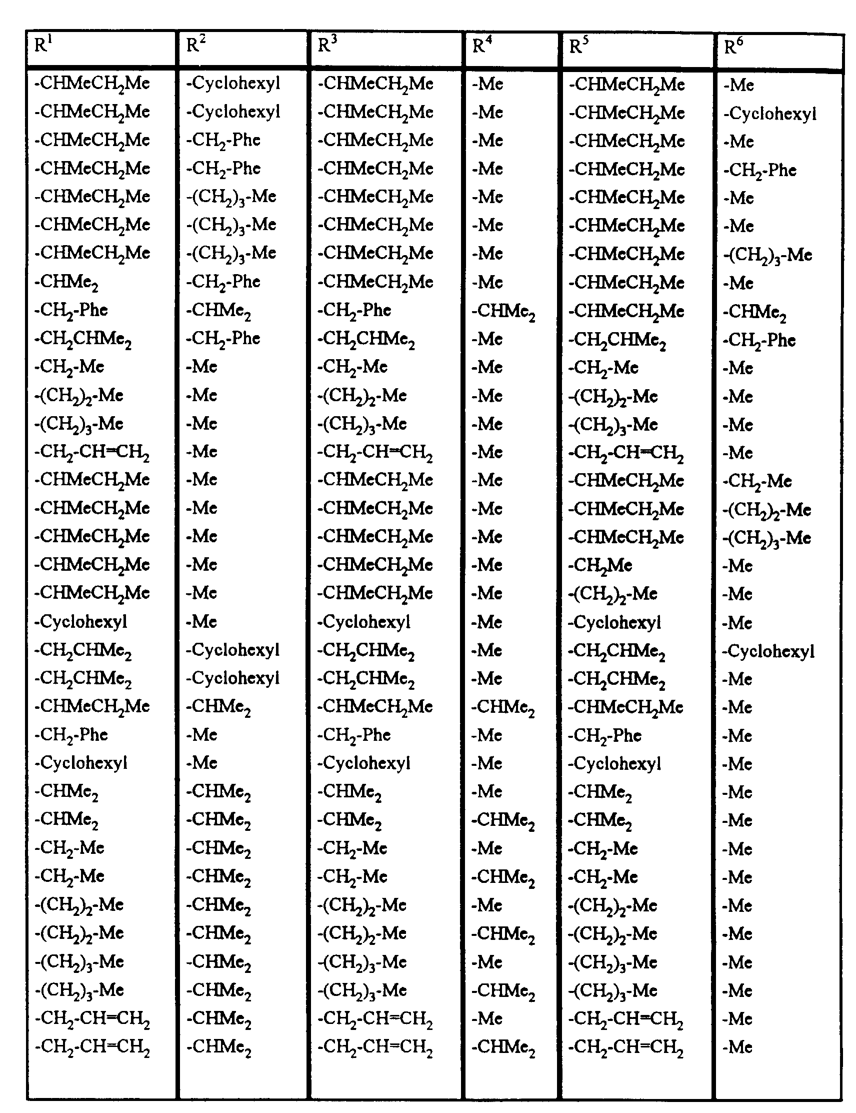

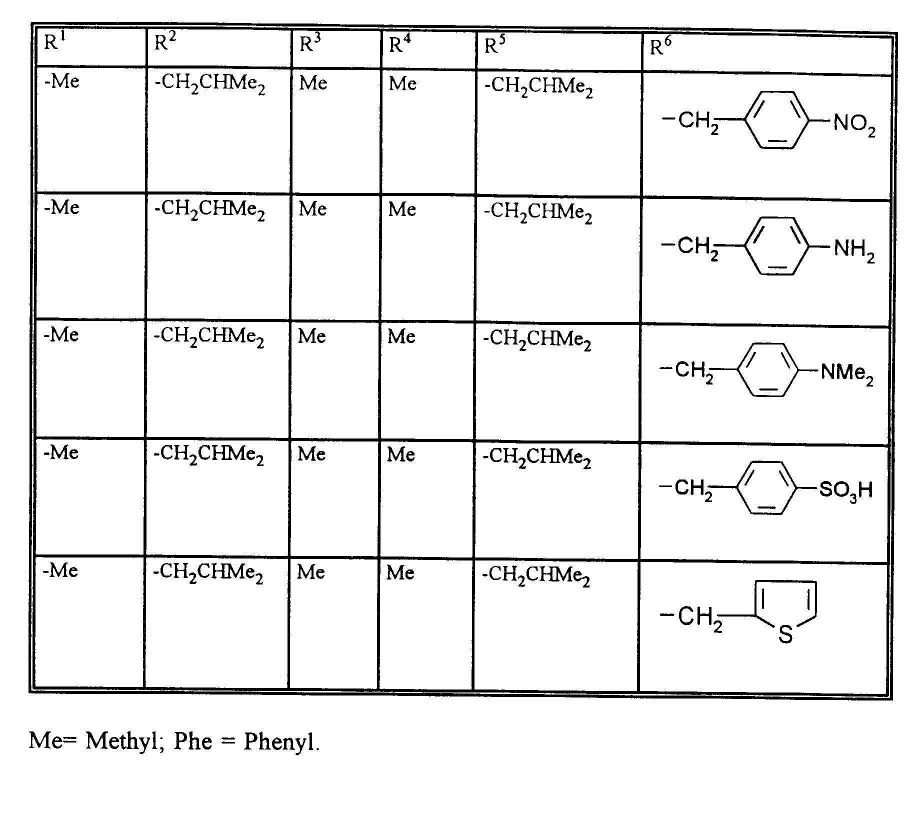

Bei Verfahren 3 werden Tetradepsipeptide in Gegenwart von Verdünnungsmitteln und geeigneten Kopplungsreagenzien cyclisiert.

Als Kopplungsreagenzien eignen sich alle Verbindungen die zur Knüpfung einer Aminbindung geeignet sind (vgl. z.B.: Houben-Weyl, Methoden der Organischen Chemie, Band 15/2; Bodanzky et al., Peptide Synthesis 2nd ed., Wiley u. Sons, New York 1976).

Vorzugsweise kommen folgende Methoden in Frage: Aktivestermethode mit Pentafluorphenol (PfP), N-Hydroxysuccinimid, 1-Hydroxybenzotriazol, Kopplung mit Carbodiimiden wie Dicyclohexylcarbodiimid oder N'-(3-Dimethylaminopropyl)-N-ethylcarbodiimid (EBC) sowie die Gemischte-Anhydrid-Methode oder die Kopplung mit Phosphoniumreagenzien, wie Benzotriazol-1-yl-oxy-tris(dimethylaminophosphonium)-hexafluorphosphat (BOP), Bis-(2-oxo-3-oxazolidinyl)-phosphoniumsäurechlorid (BOP-Cl) oder mit Phosphonsäureesterreagenzien wie Cyanphosphoniumsäurediethylester (DEPC) und Diphenylphosphorylazid (DPPA).

Besonders bevorzugt ist die Kopplung mit Bis(2-oxo-3-oxazolidinyl)-phosphoniumsäurechlorid (BOP-Cl) und N'-(3-Dimethylaminopropyl)-N-ethylcarbodiimid (EDC) in Gegenwart von 1-Hydroxybenzotriazol (HOBt).

Die Umsetzung erfolgt bei Temperaturen von 0 bis 150°C, bevorzugt bei 20 bis 100°C, besonders bevorzugt bei Raumtemperatur.

Als Verdünnungsmittel kommen alle inerten organischen Lösungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile wie z.B. Acetonitril und Propionitril, Benzonitril, Glutarsäuredinitril, darüber hinaus Amide wie z.B. Dimethylformamid, Dimethylacetamid und N-Methylpyrrolidon, sowie Dimethylsulfoxid, Tetramethylensulfon und Hexamethylphosphorsäuretriamid.

Die Cyclisierung wird in Gegenwart einer Base durchgeführt.

Als Basen kommen anorganische und organische Basen in Frage. Als Basen seien genannt: Alkali- und Erdalkalihydroxide, -carbonate, -hydrogencarbonate, -alkoholate, ferner Amine wie insbesondere tertiäre Amine, z.B. Trimethylamin, Triethylamin, N-Methylmorpholin, Pyridin, Picoline, N-Ethylpyrrolidin, Diazabicyclo-(4,3,0)-undecen (DBU), 1,4-Diazabicyclo(2,2,2)octan (DABCO), Diazabicyclo-(3,2,0)nonen (DBN), Ethyl-diisopropylamin.

Die Verbindungen der Formeln (II) und die Basen werden werden im Verhältnis 1:1 bis 1:1,5 zueinander eingesetzt. Bevorzugt ist ein etwa äquimolares Verhältnis.

Nach erfolgter Umsetzung wird das Verdünnungsmittel abdestilliert und die Verbindungen der Formel (I) in üblicher Weise, z.B. chromatographisch, gereinigt.

Nach diesen Reaktionsbedingungen wird auch bei Durchführung der Verfahren 11, 17 und 19 gearbeitet.

Die als Ausgangsverbindungen verwendeten, Tetradepsipeptide der Formel (II) können nach an sich bekannten Verfahren hergestellt werden, beispielsweise wie es von H.-G. Lerchen und H. Kunz (Tetrahedron Lett. 26 (43) (1985) S. 5257-5260; 28 (17) (1987) S. 1873-1876) unter Ausnutzung der Veresterungsmethode nach B. F. Gisin (Helv. Chim. Acta 56 (1973) S. 1476) beschrieben ist.

Die als Ausgangsmaterialien verwendeten Aminosäuren und 2-Halogen-carbonsäurederivate sind teilweise bekannt (vgl. z.B.: N-Methyl-aminosäuren: R. Bowmann et al. J. Chem. Soc. (1950) S. 1346; J. R. McDermott et al. Can. J. Chem. 51 (1973) S. 1915; H. Wurziger et al., Kontakte (Merck.Darmstadt) 3 (1987) S. 8; 2-Halogencarbonsäurederivate: S. M. Birnbaum et al. J. Amer. Chem. Soc. 76 (1954) S. 6054, C. S. Rondestvedt, Jr. et al. Org. Reactions 11 (1960) S. 189 [Review]) bzw. können nach den dort beschriebenen Verfahren erhalten werden.

Für die Kupplungsreaktion zur Darstellung der als Ausgangsverbindungen eingesetzten Depsipeptide der Formeln (II), (V), (VIII= und (IX) finden die bei Verfahren 3 genannten Kupplungsreagenzien Verwendung.

Die offenkettigen Tetradepsipeptide der Formel (II) können nach einem Verfahren erhalten werden werden, das die folgenden aufeinander folgenden Stufen umfaßt:
a) Synthese der Didepsipeptide der Formeln (VIII) und (IX) nach Verfahren 17 und 19: 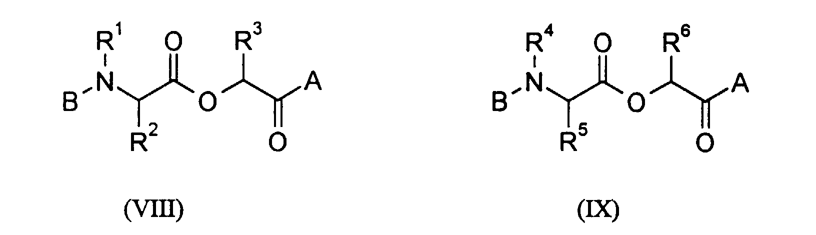
worin B eine N-terminale Schutzgruppe, wie z.B. die Benzyl oder Benzyloxycarbonylgruppe und A eine C-terminale Schutzgruppe, wie z.B. die tert-Butoxygruppe, darstellt.

Dies entspricht für Formel (VIII) beispielsweise dem folgenden Reaktionsschema: 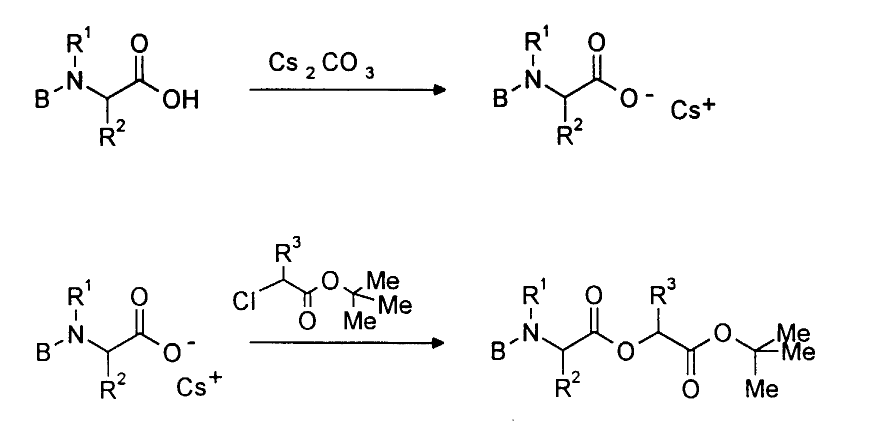 Die Herstellung der erfindungsgemäßen, enantiomerenreinen Verbindungen der Formeln (VIII) und (IX) kann gegebenenfalls auch über die Trennung der Diastereomere nach üblichen Methoden wie beispielsweise Kristallisation, durch Säulenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen.

Am Ende dieser Stufe kann eine Entfernung der N-terminalen Schutzgruppe aus den Verbindungen der Formel (IX) in an sich bekannter Weise durchgeführt werden, beispielsweise durch katalytische Hydrierung, zur Herstellung der Derivate der Formel (VII). In an sich bekannter Weise kann eine Abspaltung der C-terminalen Schutzgruppe aus den Derivaten der Formel (VIII) zur Darstellung der Verbindungen der Formel (VI) erfolgen.

Aus den Didepsipeptiden der Formeln (VI) und (VII) erfolgt die Synthese der Tetradepsipeptide der Formel (V) nach der folgenden Reaktionsgleichung (Verfahren 11): 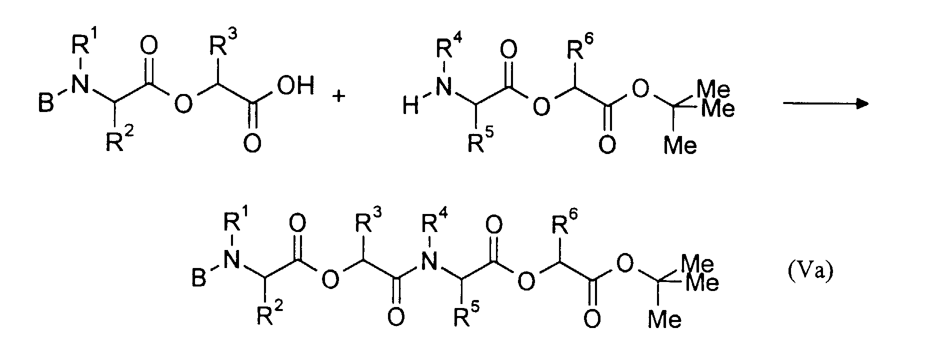 Man kann anschließend eine Entfernung der N-terminalen Schutzgruppe aus den Verbindungen der Formel (Va) durchführen, beispielsweise durch katalytische Hydrierung wie oben angegeben, zur Herstellung der Verbindungen der Formel (III) oder durch Entfernung der C-terminalen Schutzgruppe aus den Verbindungen der Formel (V) durch Verseifung zu Verbindungen der Formel (IV).

Die Abspaltung der N-terminalen Schutzgruppen durch Hydrogenolyse in den Verfahren 5b), 7 und 15 wird besonders bevorzugt mit Hydrierungsmitteln wie Wasserstoff in Gegenwart der üblichen Hydrierungskatalysatoren, wie z.B. Raney-Nickel, Palladium und Platin, durchgeführt.

Das Verfahren wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Methyl-tert.-butylether, Glykoldimethylether und Diglykoldimethylether, Tetrahydrofuran und Dioxan, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid; ferner auch Alkohole wie Methanol, Ethanol, Propanol, Isopropanol, Butanol, Isobutanol, sec.-Butanol, tert.-Butanol, Pentanol, Isopentanol, sec.-Pentanol und tert.-Pentanol sowie auch Wasser.

Die Reaktionstemperaturen können bei dem erfindungsgemäßen Verfahren in einem größeren Bereich variiert werden. Im allgemeinen arbeitet man bei Temperaturen zwischen -20°C und +200°C, vorzugsweise bei Temperaturen zwischen 0°C und 120°C.

Das Verfahren wird im allgemeinen unter Normaldruck durchgeführt. Es ist jedoch auch möglich, unter erhöhtem, im allgemeinen zwischen 10 und 100 bar, zu arbeiten.

Die Abspaltung der C-terminalen Schutzgruppen durch Verseifung in den Verfahren 5a), 9 und 13 wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Die Reaktion wird in Gegenwart von anorganischen oder organischen Protonensäuren durchgeführt. Als solche seien z.B. genannt: Salzsäure, Schwefelsäure, Trifluoressigsäure, Essigsäure, Ameisensäure.

Die Umsetzung erfolgt bei Temperaturen zwischen -20 und +50°C, vorzugsweise zwischen -10 und +20°C bei Normaldruck oder erhöhtem Druck. Es wird bevorzugt bei Normaldruck gearbeitet.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Paralilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: PHysiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl /Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

Haemonchus contortus / Schaf
Experimentell mit Haemonchus contortus infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 10 |
| 2 | 10 |
| 4 | 10 |
| 5 | 10 |
| 11 | 10 |

### Herstellungsbeispiele

1) Herstellung der Verbindungen der Formel (I) gemäß Verfahren 2
Zu einer Lösung der Verbindung II (0,104 mmol) und Hünig-Base (0,258 mmol) in Dichlormethan (100 ml) wurde bei 0°C BOP-Cl (0,124 mmol) zugegeben und 24 Stunden bei Zimmertemperatur nachgerührt. Nach dieser Zeit wurden dieselben Mengen BOP-Cl und Base zugesetzt und weitere 24 Stunden gerührt. Die Lösung wurde zweimal mit gesättigter Natriumhydrogencarbonatlösung gewaschen, über Natriumsulfat getrocknet und eingeengt. Der Rückstand wurde säulenchromatographisch mit dem Laufmittel Toluol-Ethylacetat 5:1 gereinigt.
Es wurden Verbindungen der Formel (I) erhalten, in welcher die Substituenten die folgende Bedeutung haben: 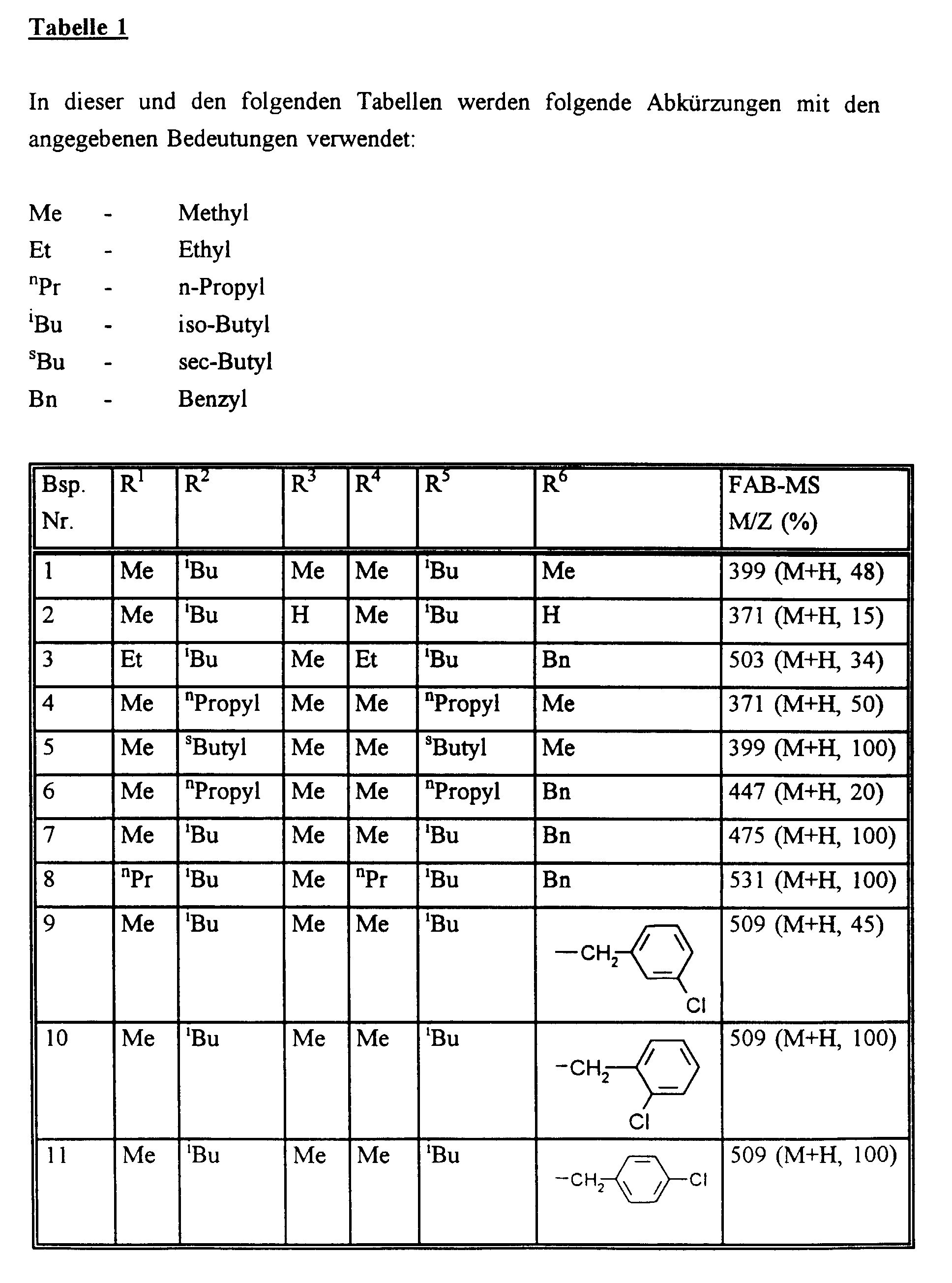
2a) Herstellung der Verbindungen der Formel (II) gemäß Verfahren 5a
In eine Lösung des tert.-Butylesters der Formel (III) (1,61 mmol) in Dichlormethan (40 ml) wurde bei 0°C 1,5 h HCl-Gas eingeleitet. Anschließend wurde auf Raumtemperatur aufgewärmt und 12 h nachgerührt. Die Lösung wurde einrotiert und im Hochvakuum getrocknet. Der in Wasser gelöste Rückstand wurde in eine Suspension eines basischen Ionenaustauschers (0,60 g) in 5 ml Wasser eingetropft, 3 h gerührt, abfiltriert und eingeengt. Nach Trocknung im Hochvakuum wurde das Produkt ohne weitere Reinigung umgesetzt.
Gemäß dieser Vorschrift wurden Verbindungen der Formel (II) erhalten, in welcher die Substituenten die folgende Bedeutung haben: 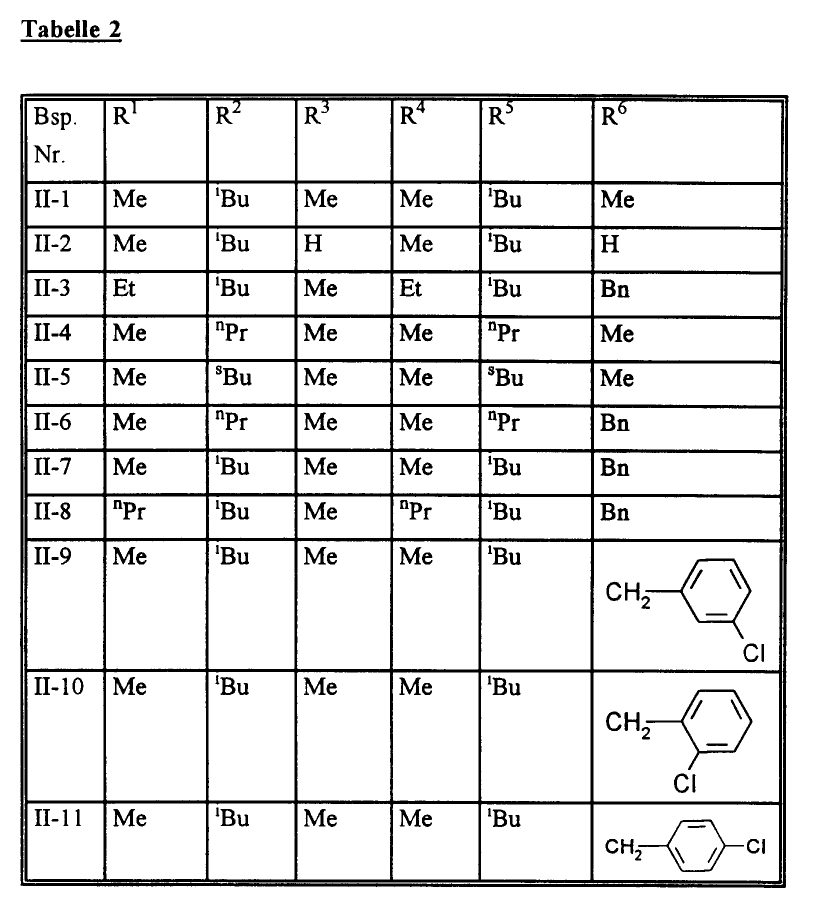
2b) Herstellung der Verbindungen der Formel (II) gemäß Verfahren 5b
Eine Lösung einer Verbindung der Formel (IV) (1,22 mmol) in Dioxan (50 ml) wurde in Gegenwart von Pd(OH)₂/C (20 %; 200 mg) bis zur Beendigung der Wasserstoffaufnahme hydriert (ca. 2 Stunden). Nach Abfiltrieren des Katalysators fiel Verbindung (II) in nahezu quantitativer Ausbeute an, die ohne zusätzliche Reinigung weiter umgesetzt wurde.
3) Herstellung der Verbindungen der Formel (III) gemäß Verfahren 7
Eine Lösung einer Verbindung der Formel (V) (1,22 mmol) in Dioxan (50 ml) wurde in Gegenwart von Pd(OH)₂/C (20 %; 200 mg) bis zur Beendigung der Wasserstoffaufnahme hydriert (ca. 2 Stunden). Nach Abfiltrieren des Katalysators fiel Verbindung (III) in nahezu quantitativer Ausbeute an, die ohne zusätzliche Reinigung weiter umgesetzt.
Gemäß dieser Vorschrift wurden Verbindungen der Formel (III) erhalten, in welcher die Substituenten die folgende Bedeutung haben: 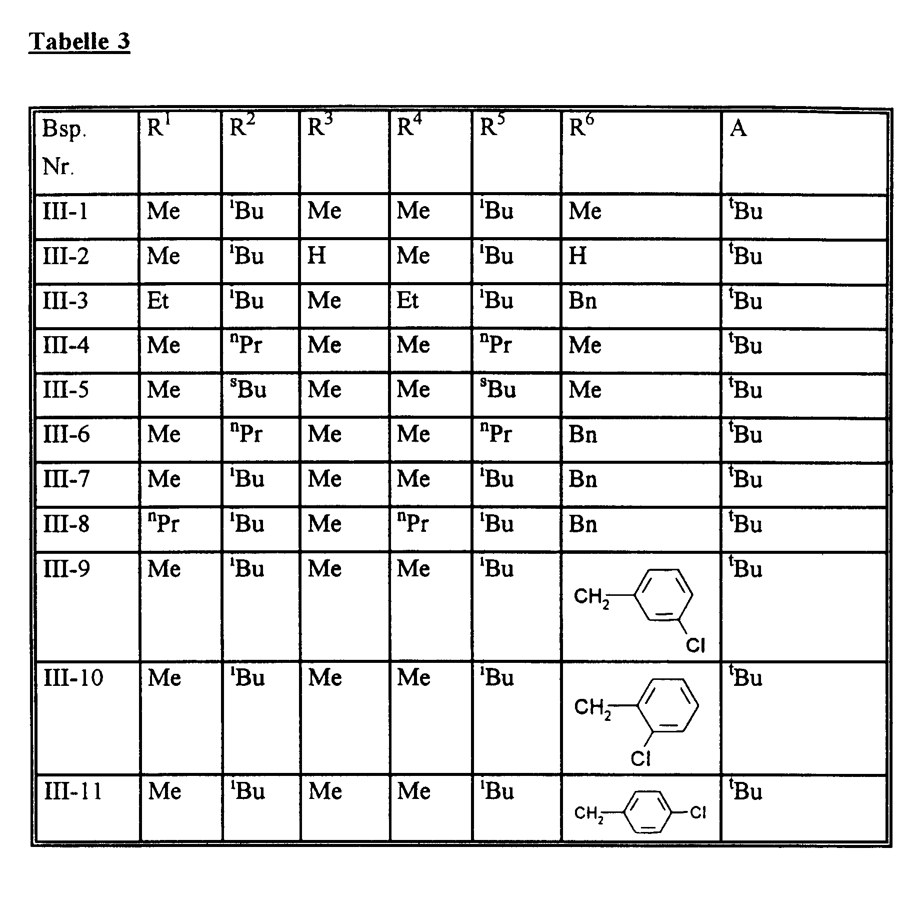
4. Herstellung der Verbindungen der Formel (IV) gemäß Verfahren 9
In eine Lösung des tert.-Butylester der Formel (V) (1,70 mmol) in Dichlormethan (40 ml) wurde bei 0°C 1,5 h HCl-Gas eingeleitet. Anschließend wurde auf Raumtemperatur aufgewärmt und 12 h nachgerührt. Die Lösung wurde einrotiert und im Hochvakuum getrocknet. Der in Wasser gelöste Rückstand wurde in eine Suspension eines basischen Ionenaustauschers (0,60 g) in 5 ml Wasser eingetropft, 3 h gerührt, abfiltriert und eingeengt. Nach Trocknung im Hochvakuum wurde das Produkt ohne weitere Reinigung umgesetzt.
Gemäß dieser Vorschrift wurden Verbindungen der Formel (IV) erhalten, in welcher die Substituenten die folgende Bedeutung haben: 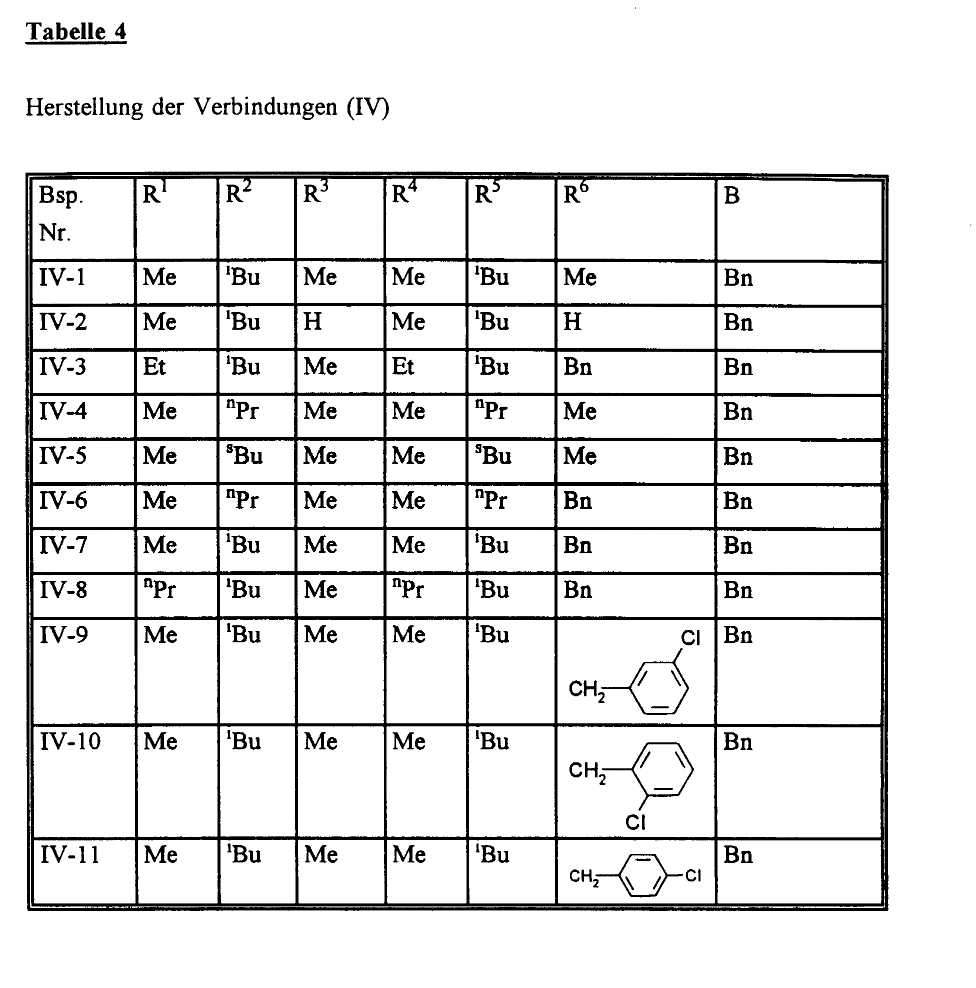
5. Herstellung der Verbindungen der Formel (V) gemäß Verfahren 11
Didepsipeptide der Formel (VI) (2,50 mmol) wurde in Dichlormethan (15 ml) vorgelegt und die auf 0°C gekühlte Lösung mit Ethyldiisopropylamin (0,91 mmol) und BoP-Cl (0,44 mmol) versetzt. Didepsipeptide der Formel (VII) (2,50 mmol) wurde in Dichlormethan (15 ml) dazu zugetropft. Es wurde 3 Stunden bei 0°C und 18 Stunden bei Raumtemperatur nachgerührt; dann mit Dichlormethan verdünnt, nacheinander mit 2N Salzsäure, gesättigter Natriumhydrogencarbonat-Lösung gewaschen, über Natriumsulfat getrocknet und eingeengt. Verbindungen der Formel (V) wurden ohne zusätzliche Reinigung weiter umgesetzt.
Gemäß dieser Vorschrift werden die Verbindungen der Formel (V) erhalten, die in der nachfolgenden Tabelle 5 zusammengefaßt sind: 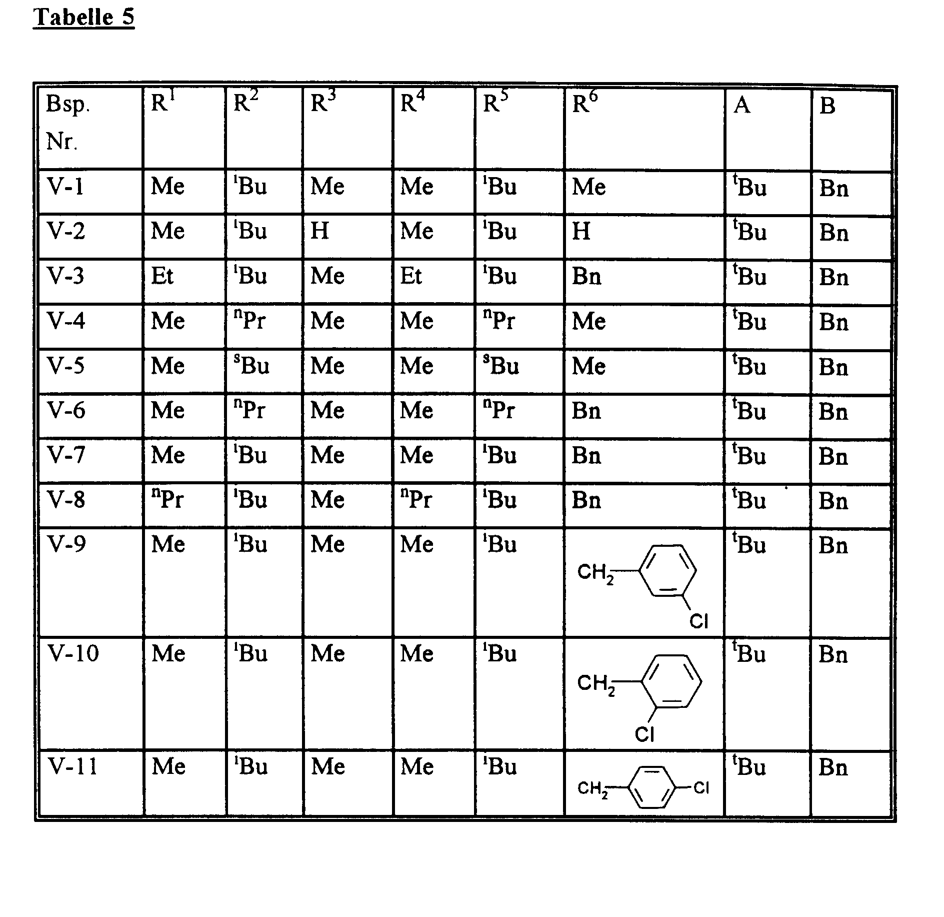
6. Herstellung der Verbindungen der Formel (VI) gemäß Verfahren 13
In eine Lösung des Didepsipeptids der Formel (VIII) (2,90 mmol) in Dichlormethan (50 ml) wurde bei 0°C HCl-Gas 2 Stunden eingeleitet.
Anschließend wurde 8 Stunden bei Raumtemperatur nachgerührt, eingeengt und im Hochvakuum getrocknet. Der in Wasser gelöste Rückstand wurde in eine Suspension eines basischen Ionenaustauschers (1,10 g) in 10 ml Wasser eingetropft, 3 Stunden gerührt, abfiltriert und eingeengt. Nach Trocknung im Hochvakuum wurde das Produkt ohne zusätzliche Reinigung weiter umgesetzt.
Gemäß dieser Vorschrift wurden die folgenden Verbindungen der Formel (VI) erhalten, in welcher die Substituenten die folgende Bedeutung haben:

**Tabelle 6**

| Bsp. Nr. | R¹ | R² | R³ | Z | B |
|---|---|---|---|---|---|
| VI-1 | Me | ⁱBu | Me | OH | Bn |
| VI-2 | Me | ⁱBu | H | OH | Bn |
| VI-3 | Et | ⁱBu | Me | OH | Bn |
| VI-4 | Me | ⁿPr | Me | OH | Bn |
| VI-5 | Me | ^{s}Bu | Me | OH | Bn |
| VI-6 | ⁿPr | ⁱBu | Me | OH | Bn |

7. Herstellung der Verbindungen der Formel (VII) gemäß Verfahren 15
Eine Lösung einer Verbindung der Formel (IX) (9,50 mmol) in Dioxan (50 ml) wurde in Gegenwart von Pd(OH)₂/C (20 %; 600 mg) bis zur Beendigung der Wasserstoffaufnahme hydriert (ca. 2 Stunden). Nach Abfiltrieren des Katalysators fiel Verbindung (VII) in nahezu quantitativer Ausbeute an, die ohne zusätzliche Reinigung weiter umgesetzt wurde.
Gemäß dieser Vorschrift wurden Verbindungen der Formel (VII) erhalten, in welcher die Substituenten die folgende Bedeutung haben: 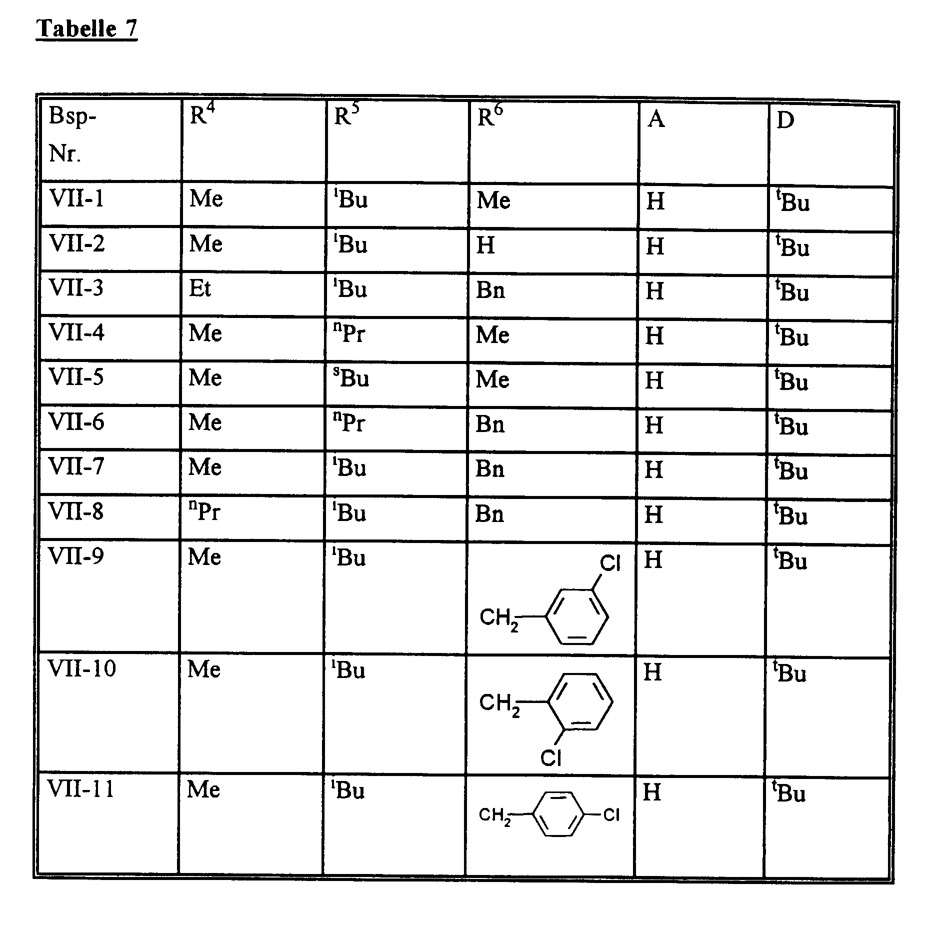
8. Herstellung der Verbindungen der Formel (VIII) gemäß Verfahren 17
Die Aminosäure der Formel (X) (0,40 mol) wurde in 1400 ml Ethanol und 800 ml Wasser gelöst, mit einer 20 %igen Caesiumcarbonatlösung (390 ml) versetzt und 2 Stunden bei Raumtemperatur gerührt. Anschließend wurde eingeengt, in Wasser (2000 ml) gelöst und gefriergetrocknet. 0,40 mol dieses Caesiumsalzes wurden in 1000 ml Dimethylformamid vorgelegt, bei Raumtemperatur mit 0,40 mol der Chlorcarbonsäure der Formel (XI) versetzt und 20 Stunden bei Raumtemperatur gerührt. Die Lösung wurde eingeengt, der Rückstand in Wasser (1000 ml) eingegossen, viermal mit Ethylacetat extrahiert, über Natriumsulfat getrocknet und eingeengt. Der Rückstand wurde ohne zusätzliche Reinigung weiter umgesetzt.
Analog wurden die Verbindungen der Formel (VIII) erhalten, in welcher die Substituenten die folgende Bedeutung haben:

**Tabelle 8**

| Bsp. Nr. | R¹ | R² | R³ | A | B |
|---|---|---|---|---|---|
| VIII-1 | Me | ⁱBu | Me | ^{t}Bu | Bn |
| VIII-2 | Me | ⁱBu | H | ^{t}Bu | Bn |
| VIII-3 | Et | ⁱBu | Me | ^{t}Bu | Bn |
| VIII-4 | Me | ⁿPr | Me | ^{t}Bu | Bn |
| VIII-5 | Me | ^{s}Bu | Me | ^{t}Bu | Bn |
| VIII-6 | ⁿPr | ⁱBu | Me | ^{t}Bu | Bn |

9. Herstellung der Verbindungen der Formel (IX) gemäß Verfahren 19
Die Aminosäure der Formel (XII) (0,40 mol) wurde in 1400 ml Ethanol und 800 ml Wasser gelöst, mit einer 20 %igen Caesiumcarbonatlösung (390 ml) versetzt und 2 Stunden bei Raumtemperatur gerührt. Anschließend wurde eingeengt, in Wasser (2000 ml) gelöst und gefriergetrocknet. 0,40 ml dieses Caesiumsalzes wurden in 100 ml Dimethylformamid vorgelegt, bei Raumtemperatur mit 0,40 ml der Chlorcarbonsäure der Formel (XIII) versetzt und 20 Stunden bei Raumtemperatur gerührt. Die Lösung wurde eingeengt, der Rückstand in Wasser (1000 ml) eingegossen, viermal mit Ethylacetat extrahiert, über Natriumsulfat getrocknet und eingeengt. Der Rückstand wurde ohne zusätzliche Reinigung weiter umgesetzt.
Analog wurden die Verbindungen der Formel (IX) erhalten, in welcher die Substituenten die folgende Bedeutung haben.

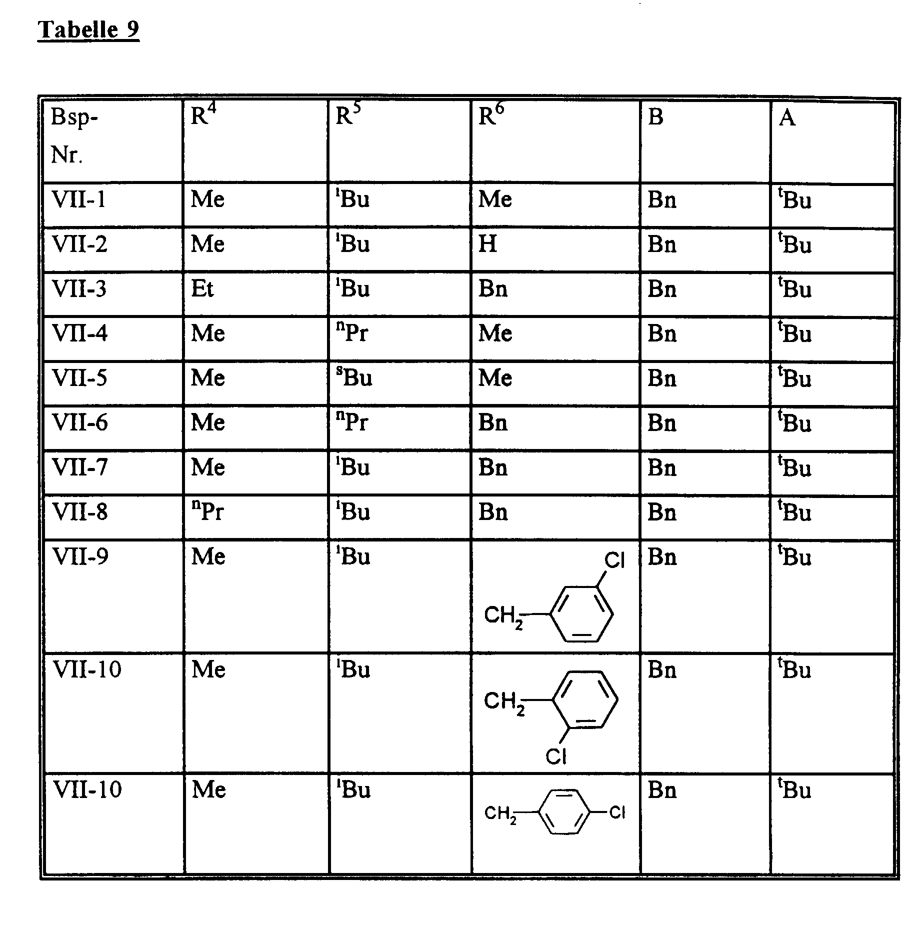

## Patentansprüche

1. Verwendung von cyclischen Depsipeptiden mit 12 Ringatomen der allgemeinen Formel (I) 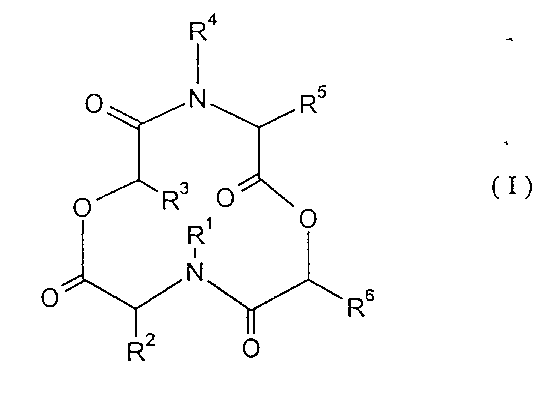 in welcher die Reste die folgende Bedeutung haben
R¹ und R⁴ stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-alkyl, Phenyl-C₁-C₄-alkyloxy-C₁-C₆-alkyl, Mercapto-C₁-C₆-alkyl, C₁-C₄-Alkylthio-C₁-C₆-alkyl, C₁-C₄-Alkylsulfinyl-C₁-C₆-alkyl, C₁-C₄-Alkylsulfonyl-C₁-C₆-alkyl, Carboxy-C₁-C₆-alykl C₁-C₄-Alkoxycarbonyl-C₁-C₆-alkyl, C₁-C₄-Phenylalkoxycarbonyl-C₁-C₆, Carbamoyl-C₁-C₆-alkyl, Amino-C₁-C₆-alkyl, C₁-C₄-Alkylamino-C₁-C₆-alkyl,C₁-C₄-Dialkylamino C₁-C₆-alkyl, Guanido-C₁-C₆-alkyl, C₁-C₄-Alkoxycarbonylamino-C₁-C₆-alkyl, 9-Fluorenylmethoxycarbonyl (Fmoc )amino-C₁-C₆-alkyl, C₃-C₇-Cycloalkyl, C₃-C₇-Cycloalkyl-C₁-C₄-alkyl, Phenyl, Phenyl-C₁-C₄-alkyl, die gegebenenfalls durch Reste aus der Reihe Halogen, Hydroxy, Nitro, CN, C₁-C₄-Alkoxy, C₁-C₄-Alkyl, substituiert sein können;
R², R³, R⁵ und R⁶ stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-alkyl, Phenyl-C₁-C₄-alkyloxy-C₁-C₆-alkyl, Mercapto- C₁-C₆-alkyl, C₁-C₄-Alkylthio-C₁-C₆-alkyl, C₁-C₄-Alkylthio-C₁-C₆-alkyl, C₁-C₄-Alkylsulfonyl-C₁-C₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Phenylalkoxycarbonyl-C₁-C₆-alkyl, Carbamoyl-C₁-C₆-alkyl, Amino-C₁-C₆-alkyl, C₁-C₄-Alkylamino-C₁-C₆-alkyl, C₁-C₄-Dialkylamino-C₁-C₆-alkyl, C₂-C₈-Alkenyl, C₃-C₇-Cycloalkyl, C₃-C₇-Cycloalkyl-C₁-C₄-alkyl, Phenyl-C₁-C₄-alkyl, Thienylmethyl, Thiazolylmethyl, Pyridylmethyl, die gegebenenfalls durch Reste aus der Reihe Halogen, Hydroxy, Sulfonyl (SO₃H), CN, NO₂, Amino, Di(C₁-C₄-alkyl)amino, C₁-C₄-Alkoxy, C₁-C₄-Alkyl substituiert sein können,
sowie deren optische Isomere und Racemate
zur Herstellung von Mitteln zur Bekämpfung von Endoparasiten in der Medizin und Tiermedizin.

2. Verwendung gemäß Anspruch 1 dadurch gekennzeichnet, daß Verbindungen der Formel I eingesetzt werden, in welcher die Reste die folgende Bedeutung haben
R¹ und R⁴ stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-alkyl, Phenyl-C₁-C₄-alkyloxy-C₁-C₆-alkyl, C₁-C₄-Alkoxycarbonylamino-C₁-C₆-alkyl, C₃-C₇-Cycloalkyl, C₃-C₇-Cycloalkyl-C₁-C₄-alkyl, Phenyl-C₁-C₄-alkyl, die gegebenenfalls durch einen oder mehrere gleiche oder verschiedene der in Anspruch 1 angegebenen Reste substituiert sein können;
R², R³, R⁵ und R⁶ stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, Phenyl-C₁-C₄-alkyloxy-C₁-C₆-alkyl, Carboxy-C₁-C₆-alkyl, C₁-C₄-Alkoxycarbonyl-C₁-C₆-alkyl, C₁-C₄-Phenyl-alkoxycarbonyl-C₁-C₆-alkyl, C₁-C₄-Alkylamino-C₁-C₆-alkyl, C₁-C₄-Dialkylamino-C₁-C₆-alkyl, C₂-C₈-Alkenyl, C₃-C₇-Cycloalkyl, C₃-C₇-Cycloalkyl-C₁-C₄-alkyl, Phenyl, Phenyl-C₁-C₄-alkyl, Thienylmethyl, die gegebenenfalls duchr einen oder mehrere gleiche oder verschiedene der in Anspruch 1 angegebenen Reste substituiert sein können,
sowie deren optische Isomere und Racemate.

3. Verwendung gemäß Anspruch 1 dadurch gekennzeichnet, daß Verbindungen der Formel (I) eingesetzt werden, in welcher die Reste die folgende Bedeutung haben
R¹ und R⁴ stehen unabhängig voneinander für Wasserstoff, geradkettiges oder verzweigtes C₁-C₈-Alkyl, C₃-C₇-Cycloalkyl-C₁-C₄-alkyl, Phenyl-C₁-C₄-alkyl,
R², R³, R⁵ und R⁶ stehen unabhängig voneinander für Wasserstoff, geradkettiges oder verzweigtes C₁-C₈-Alkyl, 1-5 Halogen-C₁₋₄-alkyl, C₂-C₈-Alkenyl, C₃-C₇-Cycloalkyl-C₁-C₄-alkyl, Phenyl-C₁-C₄-alkyl, Thienylmethyl,die gegebenenfalls durch einen oder mehrere gleiche oder verschiedene der in Anspruch 1 angegebenen Reste substituiert sein können,
sowie deren optische Isomere und Racemate.

4. Verwendung gemäß Anspruch 1 von Verbindungen der allgemeinen Formel (I), in welcher
Die Reste R¹ bis R⁶ die folgende Bedeutung haben:

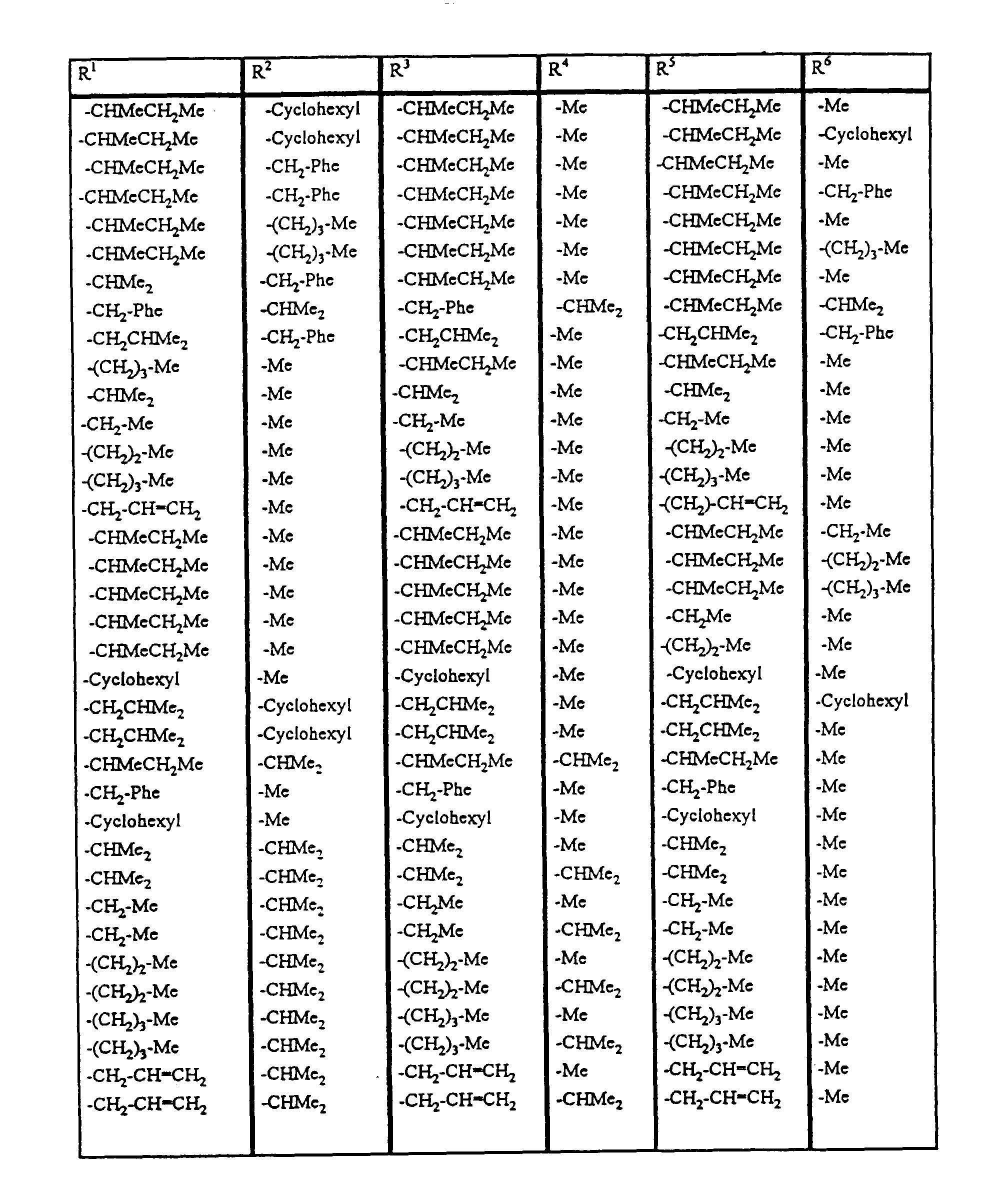

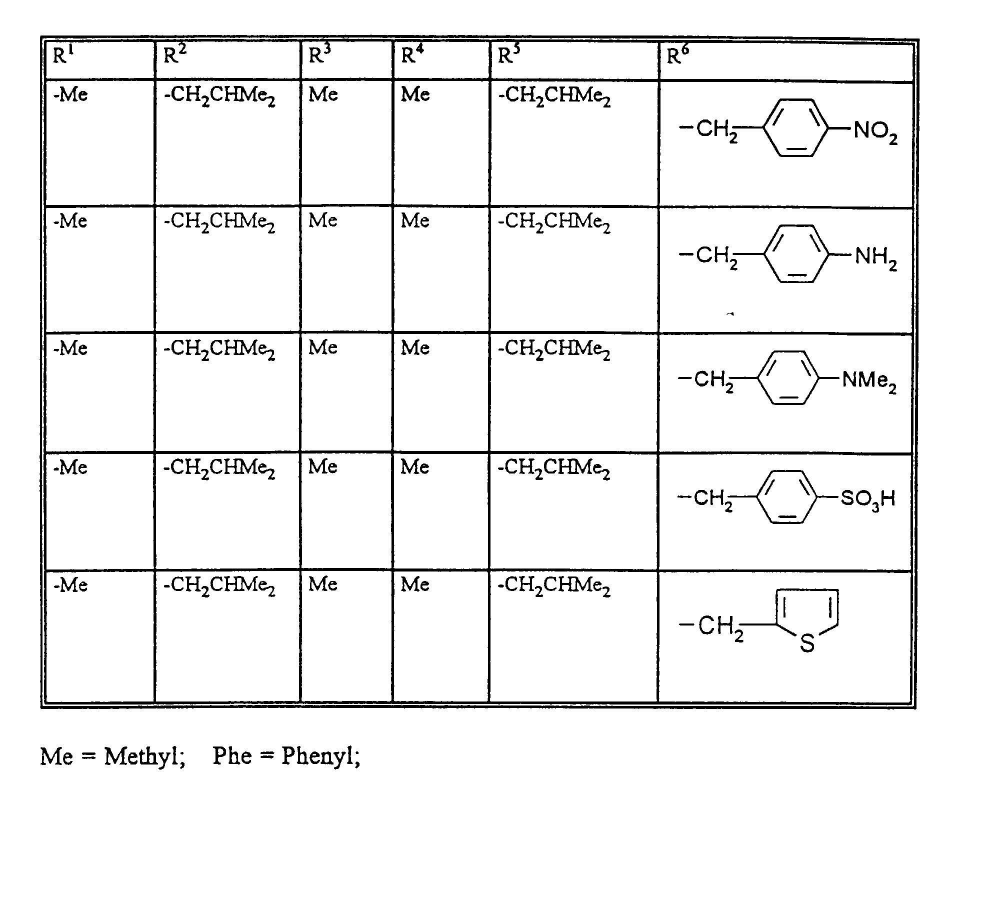

5. Endoparasitizide Mittel, gekennzeichnet durch einen Gehalt an mindestens einem cyclischen Depsipeptid mit 12 Ringatomen der Formel (I) gemäß Ansprüchen 1-4.

6. Cyclische Depsipeptide mit 12 Ringatomen der Formel (I) 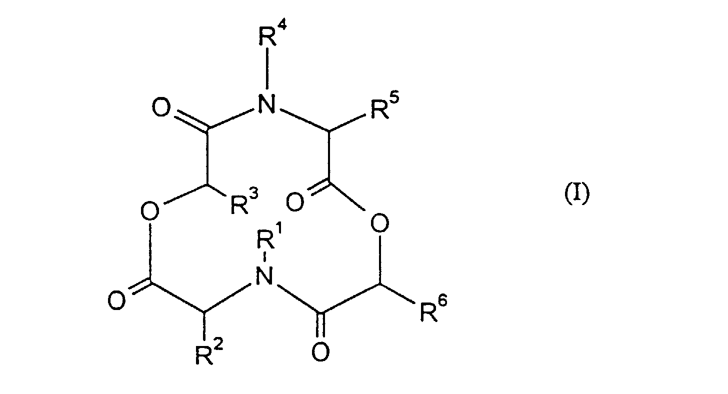 in welcher die Reste die folgende Bedeutung haben
R¹ und R⁴ stehen unabhängig voneinander für Wasserstoff, geradkettiges oder verzweigtes C₁-C₈-Alkyl, 1-5 Halogen-C₁₋₄-alkyl, C₃-C₆-Cycloalkyl, Phenyl, Phenyl-C₁-C₄-alkyl, die gegebenenfalls durch Reste aus der Reihe Halogen, Hydroxy, Nitro, CN, C₁-C₄-Alkoxy, C₁-C₄-Alkyl, substituiert sein können,
R², R³, R⁵ stehen unabhängig voneinander für Wasserstoff, geradkettiges oder verzweigtes C₁-C₈-Alkyl, 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-alkyl, Mercapto-C₁-C₆-alkyl, C₁-C₄-Alkylthio-C₁-C₆-alkyl, C₁-C₄-Alkylsulfinyl-C₁₋₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Phenylalkoxycarbonyl-C₁-C₆ -alkyl, Carbamoyl-C₁-C₆-alkyl, Amino-C₁-C₆-alkyl, C₁-C₄-Alkylamino-C₁-C₆-alkyl, C₁-C₄Dialkylamino-C₁-C₆-alkyl, C₂-C₈-Alkenyl, C₃-C₇-Cycloalkyl, C₃-C₇-Cycloalkyl-C₁-C₄-alkyl, Phenyl, Phenyl-C₁-C₄-alkyl, Thienylmethyl, Thiazolylmethyl, Pyridylmethyl, die gegebenenfalls durch Reste aus der Reihe Halogen, Hydroxy, Sulfonyl (SO₃H), CN, NO₂, Amino, Di (C₁-C₄-alkyl)amino, C₁-C₄-Alkyl, substituiert sein können;
R⁶ steht für 1-5 Halogen-C₁₋₄-alkyl, Hydroxy-C₁-C₆-alkyl, C₁-C₄-Alkanoyloxy-C₁-C₆-alkyl, C₁-C₄-Alkoxy-C₁-C₆-alkyl, Mercapto-C₁-C₆-alkyl, C₁-C₄-Alkylthio-C₁-C₆-alkyl, C₁-C₄-Alkylsulfinyl-C₁₋₆-alkyl, C₁-C₄-Alkylsulfonyl-C₁-C₆-alkyl, Carboxy-C₁-C₆-alkyl, C₁-C₄-Alkoxycarbonyl-C₁-C₆-alkyl, C₁-C₄-Phenylalkoxycarbonyl-C₁-C₆ -alkyl, Carbamoyl-C₁-C₆-alkyl, Amino-C₁-C₆-alkyl, C₁-C₄-Alkylamino-C₁-C₆-alkyl, C₁-C₄-Amino-C₁-C₆-alkyl, C₁-C₄-Alkylamino-C₁-C₆-alkyl, C₁-C₄-Dialkylamino-C₁-C₆-alkyl, C₂-C₈-Alkenyl, C₃-C₇-Cycloalkyl, C₃-C₇-Cycloalkyl-C₁-C₄-alkyl, Phenyl, Phenyl-C₁-C₄-alkyl, Thienylmethyl, Thiazolylmethyl, Pyridylmethyl, die gegebenenfalls durch Reste aus der Reihe Halogen, Hydroxy, Sulfonyl (SO₃H), CN, NO₂, Amino, Di (C₁-C₄-alkyl)amino, C₁-C₄-Alkyl, substituiert sein können;
sowie deren optische Isomere und Racemate
mit der Maßgabe für den Fall, daß einer der Reste R¹ und R⁴ für einen anderen als einen Methylrest steht, der Rest R⁶ zusätzlich zu den angegebenen Bedeutungen für Wasserstoff oder geradkettiges oder verzweigtes C₁₋₉-Alkyl stehen kann.

7. Cyclische Depsipeptide gemäß Anspruch 6, wobei in Formel (I) die Reste die folgende Bedeutung haben
R¹ und R⁴ stehen unabhängig voneinander für Wasserstoff, geradkettiges oder verzweigtes C₁-C₈-Alkyl, Phenyl-C₁-C₄-alkyl, die gegebenenfalls durch einen oder mehrere der in Anspruch 6 angegebenen Substituenten substituiert sein können;
R², R³, R⁵ stehen unabhängig voneinander für Wasserstoff, geradkettiges oder verzweigtes C₁-C₈-Alkyl, 1-5 Halogen-C₁₋₄-alkyl, C₂-C₈-Alkenyl, C₃-C₇-Cycloalkyl-C₁-C₄-alkyl, Phenyl-C₁-C₄-alkyl, Thienylmethyl, die gegebenenfalls durch einen oder mehrere gleiche oder verschiedene der in Anspruch 6 angegebenen Substituenten substituiert sein können;
R⁶ steht für 1-5 Halogen-C₁₋₄-alkyl, C₂-C₈-Alkenyl, C₃-C₇-Cycloalkyl-C₁-C₄-alkyl, Phenyl-C₁-C₄-alkyl, Thienylmethyl, die gegebenenfalls durch einen oder mehrere gleiche oder verschiedene der in Anspruch 6 angegebenen Reste substituiert sein können,
sowie deren optische Isomere und Racemate,
mit der Maßgabe für den Fall, daß einer der Reste R¹ und R⁴ für einen anderen als einen Methylrest steht, der Rest R⁶ zusätzlich zu den angegebenen Bedeutungen für Wasserstoff oder geradkettiges oder verzweigtes C₁₋₉-Alkyl stehen kann.

8. Cyclische Depsipeptide gemäß Formel (I) in Anspruch 6 wobei die Reste R¹-R⁴ die folgende Bedeutung haben

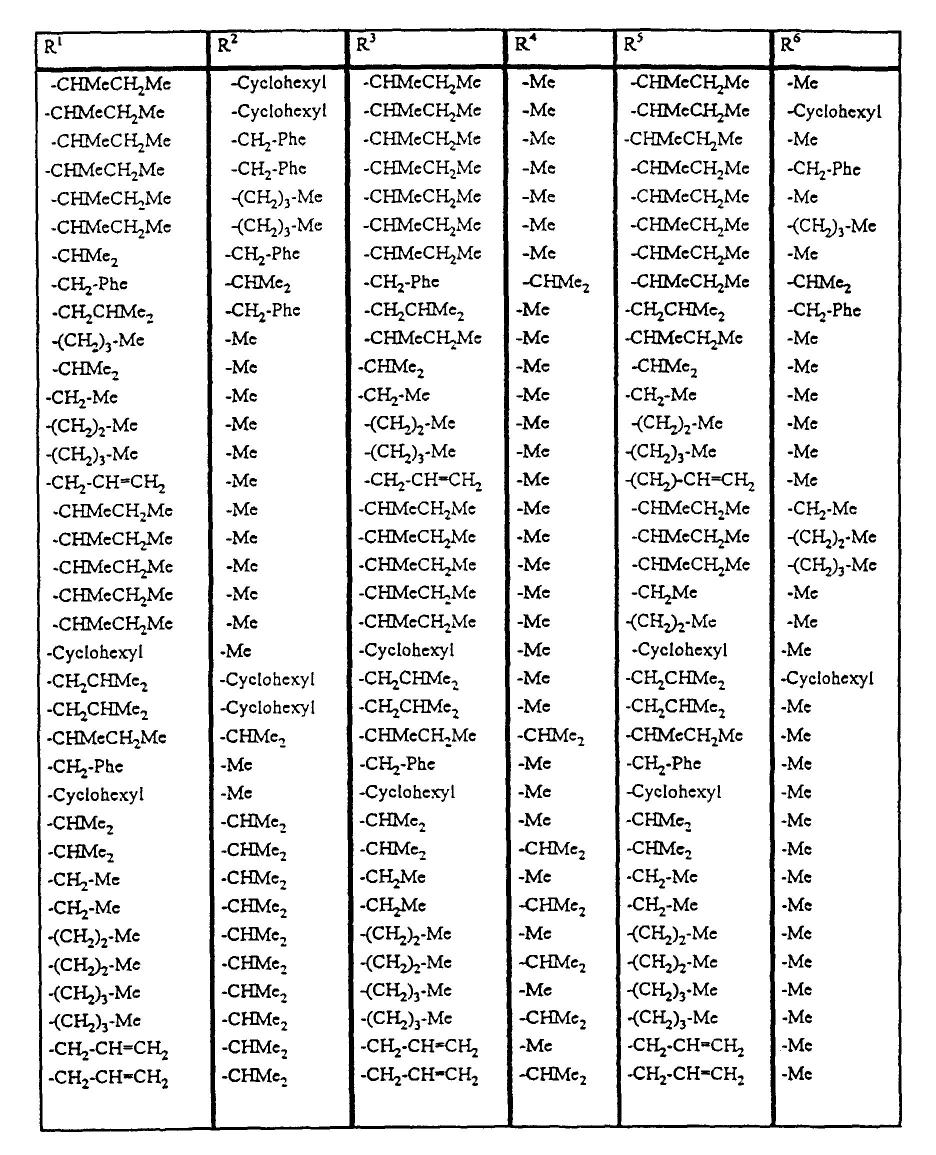

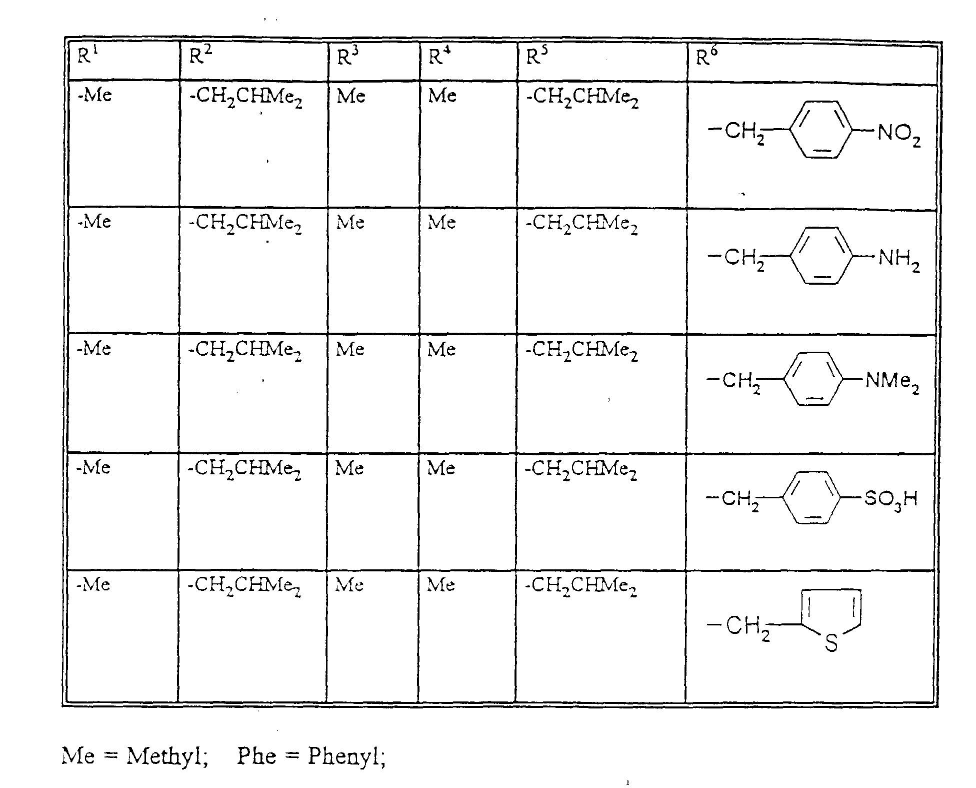

9. Verfahren zur Herstellung der neuen cyclischen Depsipeptide mit 12 Ringatomen der Formel (I) gemäß Anspruch 6 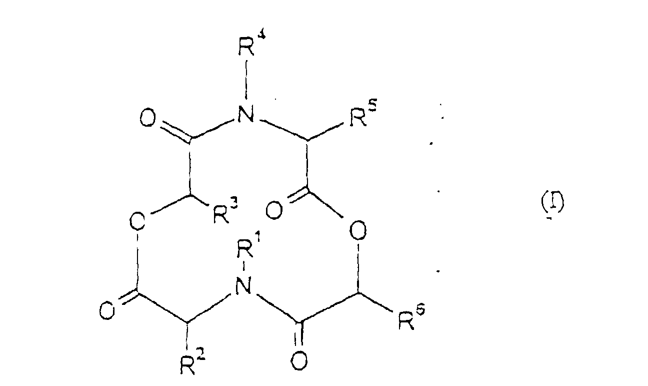 in welcher
die Reste R¹ - R⁶ die in Anspruch 6 angegebene Bedeutung haben,
dadurch gekennzeichnet, daß man offenkettige Tetradepsipeptide der Formel (II) 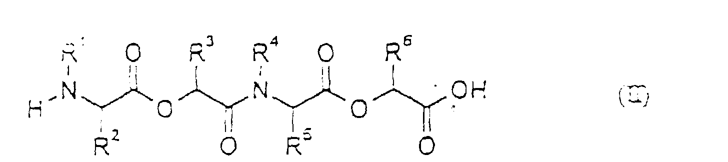 in welcher
R¹ bis R⁶ die oben angegebene Bedeutung haben,
in Gegenwart eines Verdünnungsmittel und in Gegenwart eines Kopplungsreagenzes cyclisiert.

10. Cyclische Depsipeptide mit 12 Ringatomen der Formel (I) gemäß Anspruch 6 zur Verwendung in endoparasitiziden Mitteln.

## Claims

1. Use of cyclic depsipeptides having 12 ring atoms, of the general formula (I) 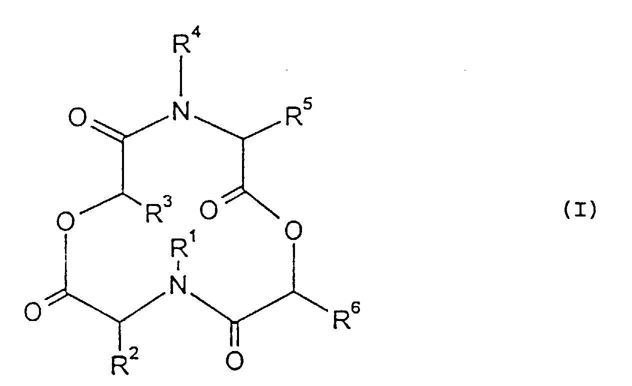 in which the radicals have the following meanings:
R¹ and R⁴ independently of one another represent hydrogen, straight-chain or branched C₁-C₈-alkyl, l-5-halogeno-C₁₋₄-alkyl, hydroxy-C₁-C₆-alkyl, C₁-C₄-alkanoyloxy-C₁-C₆-alkyl, C₁-C₄-alkoxy-C₁-C₆-alkyl, phenyl-C₁-C₄-alkyloxy-C₁-C₆-alkyl, mercapto-C₁-C₆-alkyl,C₁-C₄-alkylthio-C₁-C₆-alkyl, C₁-C₄-alkylsulphinyl-C₁-C₆-alkyl, C₁-C₄-alkylsulphonyl-C₁-C₆-alkyl, carboxy-C₁-C₆-alkyl, C₁-C₄-alkoxycarbonyl-C₁-C₆-alkyl, C₁-C₄-phenylalkoxycarbonyl-C₁-C₆-alkyl, carbamoyl-C₁-C₆-alkyl, amino-C₁-C₆-alkyl, C₁-C₄-alkylamino-C₁-C₆-alkyl, C₁-C₄-dialkylamino-C₁-C₆-alkyl, guanido-C₁-C₆-alkyl, C₁-C₄-alkoxycarbonylamino-C₁-C₆-alkyl, 9-fluorenylmethoxycarbonyl (Fmoc)amino-C₁-C₆-alkyl, C₃-C₇-cycloalkyl, C₃-C₇-cycloalkyl-C₁-C₄-alkyl, phenyl, phenyl-C₁-C₄-alkyl, it optionally being possible for these to be substituted by radicals from the series consisting of halogen, hydroxyl, nitro, CN, C₁-C₄-alkoxy and C₁-C₄-alkyl;
R², R³, R⁵ and R⁶ independently of one another represent hydrogen, straight-chain or branched C₁-C₈-alkyl, 1-5-halogeno-C₁₋₄-alkyl, hydroxy-C₁-C₆-alkyl, C₁-C₄-alkanoyloxy-C₁-C₆-alkyl, C₁-C₄-alkoxy-C₁-C₆-alkyl, phenyl-C₁-C₄-alkyloxy-C₁-C₆-alkyl, mercapto-C₁-C₆-alkyl, C₁-C₄-alkylthio-C₁-C₆-alkyl, C₁-C₄-alkylsulphinyl-C₁-C₆-alkyl, C₁-C₄-alkylsulphonyl-C₁-C₆-alkyl, carboxy-C₁-C₆-alkyl, C₁-C₄-alkoxycarbonyl-C₁-C₆-alkyl, C₁-C₄-phenylalkoxycarbonyl-C₁-C₆-alkyl, carbamoyl-C₁-C₆-alkyl, amino-C₁-C₆-alkyl, C₁-C₄-alkylamino-C₁-C₆-alkyl, C₁-C₄-dialkylamino-C₁-C₆-alkyl, C₂-C₈-alkenyl, C₃-C₇-cycloalkyl, C₃-C₇-cycloalkyl-C₁-C₄-alkyl, phenyl-C₁-C₄-alkyl, thienylmethyl, thiazolylmethyl, pyridylmethyl, it optionally being possible for these to be substituted by radicals from the series consisting of halogen, hydroxyl, sulphonyl (SO₃H), CN, NO₂, amino, di(C₁-C₄-alkyl)amino, C₁-C₄-alkoxy and C₁-C₄-alkyl,
and their optical isomers and racemates
for the preparation of endoparasiticides in medicine and veterinary medicine.

2. Use according to Claim 1, characterized in that compounds of the formula I are employed in which the radicals have the following meanings:
R¹ and R⁴ independently of one another represent hydrogen, straight-chain or branched C₁-C₈-alkyl, 1-5-halogeno-C₁₋₄-alkyl, hydroxy-C₁-C₆-alkyl, C₁-C₄-alkanoyloxy-C₁-C₆-alkyl, C₁-C₄-alkoxy-C₁-C₆-alkyl, phenyl-C₁-C₄-alkyloxy-C₁-C₆-alkyl, C₁-C₄-alkoxycarbonylamino-C₁-C₆-alkyl, C₃-C₇-cycloalkyl, C₃-C₇-cycloalkyl-C₁-C₄-alkyl, phenyl-C₁-C₄-alkyl, it optionally being possible for these to be substituted by one or more identical or different radicals from amongst those given in Claim 1;
R², R³, R⁵ and R⁶ independently of one another represent hydrogen, straight-chain or branched C₁-C₈-alkyl, 1-5-halogeno-C₁₋₄-alkyl, hydroxy-C₁-C₆-alkyl, phenyl-C₁-C₄-alkyloxy-C₁-C₆-alkyl, carboxy-C₁-C₆-alkyl, C₁-C₄-alkoxycarbonyl-C₁-C₆-alkyl, C₁-C₄-phenyl-alkoxycarbonyl-C₁-C₆-alkyl, C₁-C₄-alkylamino-C₁-C₆-alkyl, C₁-C₄-dialkylamino-C₁-C₆-alkyl, C₂-C₈-alkenyl, C₃-C₇-cycloalkyl, C₃-C₇-cycloalkyl-C₁-C₃-alkyl, phenyl, phenyl-C₁-C₄-alkyl, thienylmethyl, it optionally being possible for these to be substituted by one or more identical or different radicals from amongst those given in Claim 1,
and their optical isomers and racemates.

3. Use according to Claim 1, characterized in that compounds of the formula (I) are employed in which the radicals have the following meanings:
R¹ and R⁴ independently of one another represent hydrogen, straight-chain or branched C₁-C₈-alkyl, C₃-C₇-cycloalkyl-C₁-C₄-alkyl, phenyl-C₁-C₄-alkyl,
R², R³, R⁵ and R⁶ independently of one another represent hydrogen, straight-chain or branched C₁-C₈-alkyl, 1-5-halogeno-C₁₋₄-alkyl, C₂-C₈-alkenyl, C₃-C₇-cycloalkyl-C₁-C₄-alkyl, phenyl-C₁-C₄-alkyl, thienylmethyl, it optionally being possible for these to be substituted by one or more identical or different radicals from amongst those given in Claim 1,
and their optical isomers and racemates.

4. Use according to Claim 1 of compounds of the general formula (I) in which the radicals R¹ to R⁶ have the following meanings:

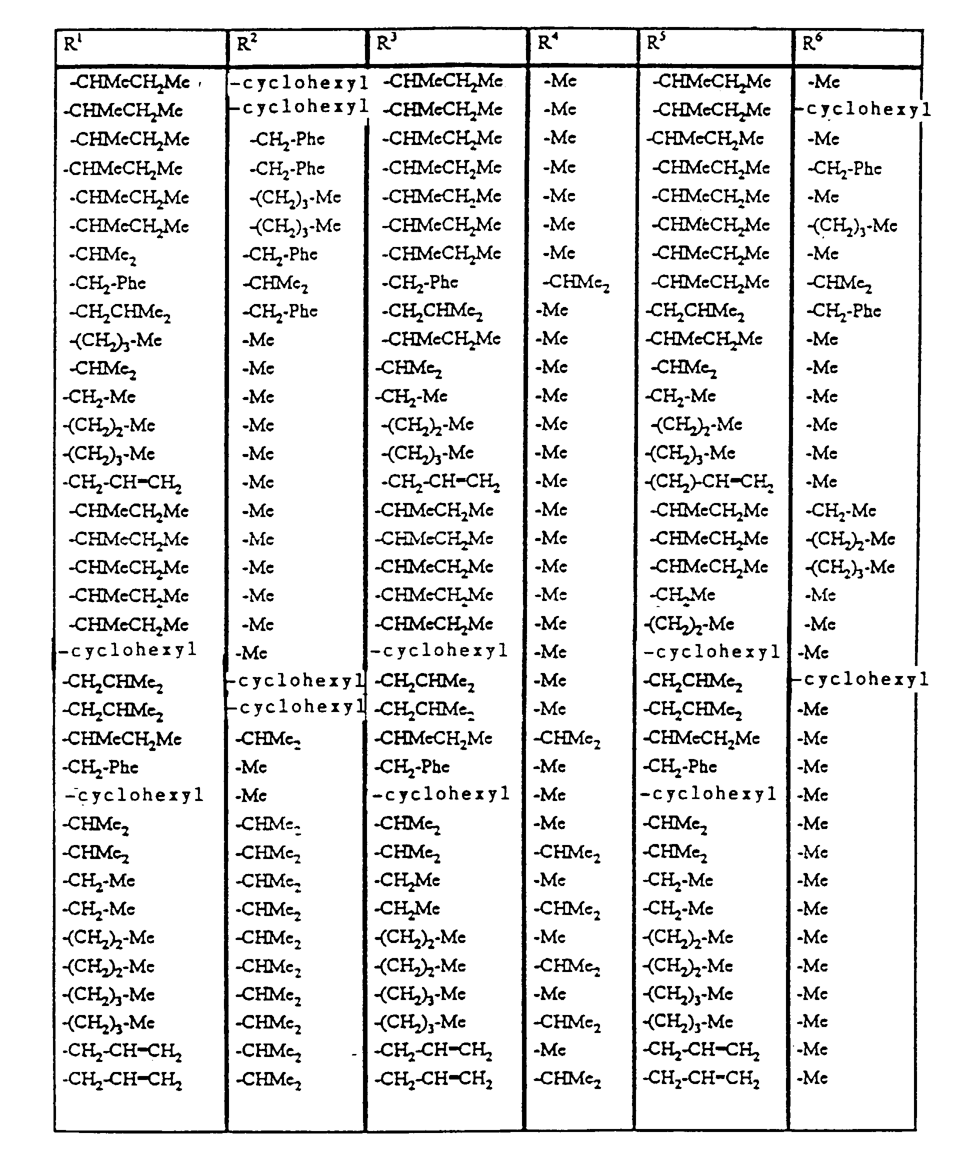

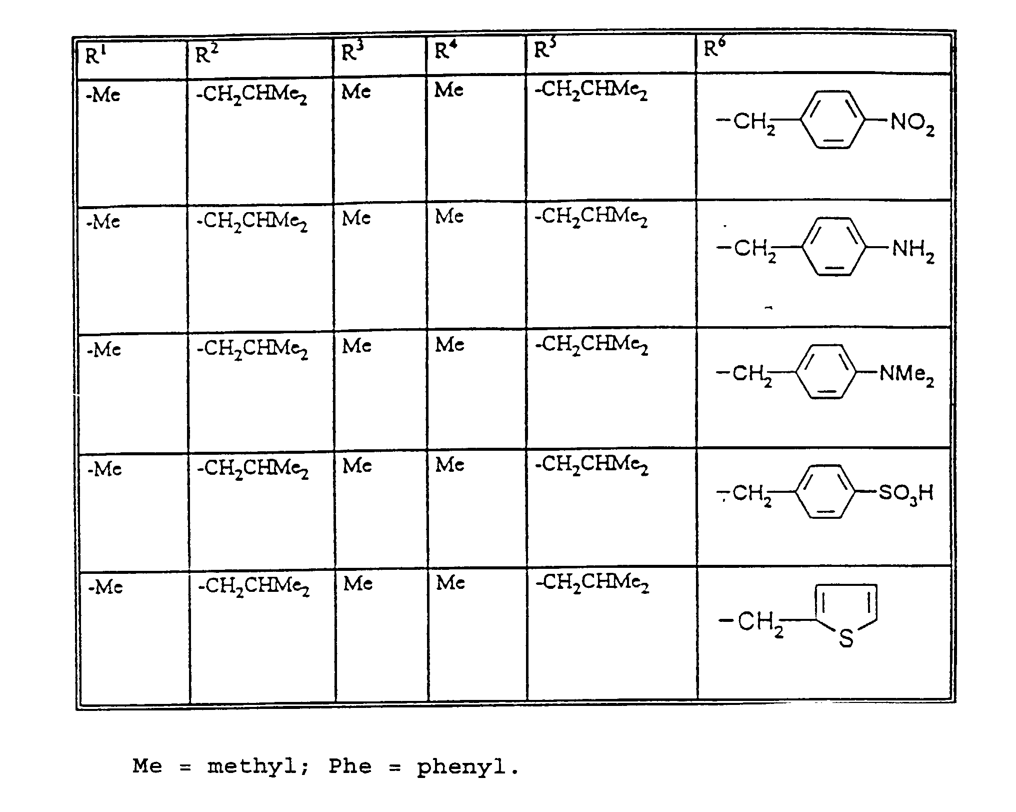

5. Endoparasiticides, characterized in that they comprise at least one cyclic depsipeptide having 12 ring atoms, of the formula (I) according to Claims 1-4.

6. Cyclic depsipeptides having 12 ring atoms, of the formula (I) 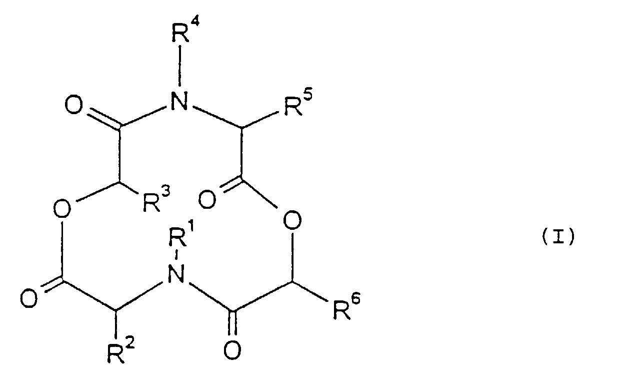 in which the radicals have the following meanings:
R¹ and R⁴ independently of one another represent hydrogen, straight-chain or branched C₁-C₈-alkyl, 1-5-halogeno-C₁₋₄-alkyl, C₃-C₇-cycloalkyl, phenyl, phenyl-C₁-C₄-alkyl, it optionally being possible for these to be substituted by radicals from the series consisting of halogen, hydroxyl, nitro, CN, C₁-C₄-alkoxy and C₁-C₄-alkyl,
R², R³, R⁵ independently of one another represent hydrogen, straight-chain or branched C₁-C₈-alkyl, 1-5-halogeno-C₁₋₄-alkyl, hydroxy-C₁-C₆-alkyl, C₁-C₄-alkanoyloxy-C₁-C₆-alkyl, C₁-C₄-alkoxy-C₁-C₆-alkyl, mercapto-C₁-C₆-alkyl, C₁-C₄-alkylthio-C₁-C₆-alkyl, C₁-C₄-alkylsulphinyl-C₁₋₆-alkyl, C₁-C₄-alkylsulphonyl-C₁-C₆-alkyl, carboxy-C₁-C₆-alkyl, C₁-C₄-alkoxycarbonyl-C₁-C₆-alkyl, C₁-C₄-phenylalkoxycarbonyl-C₁-C₆-alkyl, carbamoyl-C₁-C₆-alkyl, amino-C₁-C₆-alkyl, C₁-C₄-alkylamino-C₁-C₆-alkyl, C₁-C₄-dialkyiamino-C₁-C₆-alkyl, C₂-C₈-alkenyl, C₃-C₇-cycloalkyl-C₁-C₄-alkyl, phenyl, phenyl-C₁-C₄-alkyl, thienylmethyl, thiazolylmethyl, pyridylmethyl, it optionally being possible for these to be substituted by radicals from the series consisting of halogen, hydroxyl, sulphonyl (SO₃H), CN, NO₂, amino, di(C₁-C₄-alkyl)amino and C₁-C₄-alkyl;
R⁶ represents 1-5-halogeno-C₁₋₄-alkyl, hydroxy-C₁-C₆-alkyl, C₁-C₄-alkanoyloxy-C₁-C₆-alkyl, C₁-C₄-alkoxy-C₁-C₆-alkyl, mercapto-C₁-C₆-alkyl, C₁-C₄- alkylthio-C₁-C₆-alkyl, C₁-C₄-alkylsulphinyl-C₁₋₆-alkyl, C₁-C₄-alkylsulphonyl-C₁-C₆-alkyl, carboxy-C₁-C₆-alkyl, C₁-C₄-alkoxycarbonyl-C₁-C₆-alkyl, C₁-C₄-phenylalkoxycarbonyl-C₁-C₆-alkyl, carbamoyl-C₁-C₆-alkyl, amino-C₁-C₆-alkyl, C₁-C₄-alkylamino-C₁-C₆-alkyl, C₁-C₄-dialkylamino-C₁-C₆-alkyl, C₂-C₈-alkenyl, C₃-C₇-cycloalkyl, C₃-C₇-cycloalkyl-C₁-C₄-alkyl, phenyl, phenyl-C₁-C₄-alkyl, thienylmethyl, thiazolylmethyl, pyridylmethyl, it optionally being possible for these to be substituted by radicals from the series consisting of halogen, hydroxyl, sulphonyl (SO₃H), CN, NO₂, amino, di(C₁-C₄-alkyl)amino and C₁-C₄-alkyl;
and their optical isomers and racemates
with the proviso that, in the event that one of the radicals R¹ and R⁴ represents other than a methyl radical, the radical R⁶, in addition to the abovementioned meanings, may represent hydrogen or straight-chain or branched C₁₋₉-alkyl.

7. Cyclic depsipeptides according to Claim 6, where, in formula (I), the radicals have the following meanings:
R¹ and R⁴ independently of one another represent hydrogen, straight-chain or branched C₁-C₈-alkyl, phenyl-C₁-C₄-alkyl, it optionally being possible for these to be substituted by one or more of the substituents given in Claim 6;
R², R³, R⁵ independently of one another represent hydrogen, straight-chain or branched C₁-C₈-alkyl, 1-5-halogeno-C₁₋₄-alkyl, C₂-C₈-alkenyl, C₃-C₇-cycloalkyl-C₁-C₄-alkyl, phenyl-C₁-C₄-alkyl, thienylmethyl, it optionally being possible for these to be substituted by one or more identical or different substituents from amongst those given in Claim 6;
R⁶ represents 1-5-halogeno-C₁₋₄-alkyl, C₂-C₈-alkenyl, C₃-C₇-cycloalkyl-C₁-C₄-alkyl, phenyl-C₁-C₄-alkyl, thienylmethyl, it optionally being possible for these to be substituted by one or more identical or different radicals from amongst those given in Claim 6,
and their optical isomers and racemates,
with the proviso that, in the event that one of the radicals R¹ and R⁴ represents other than a methyl radical, the radical R⁶, in addition to the abovementioned meanings, may represent hydrogen or straight-chain or branched C₁₋₉-alkyl.

8. Cyclic depsipeptides of formula (I) in Claim 6, where the radicals R¹-R⁴ have the following meanings:

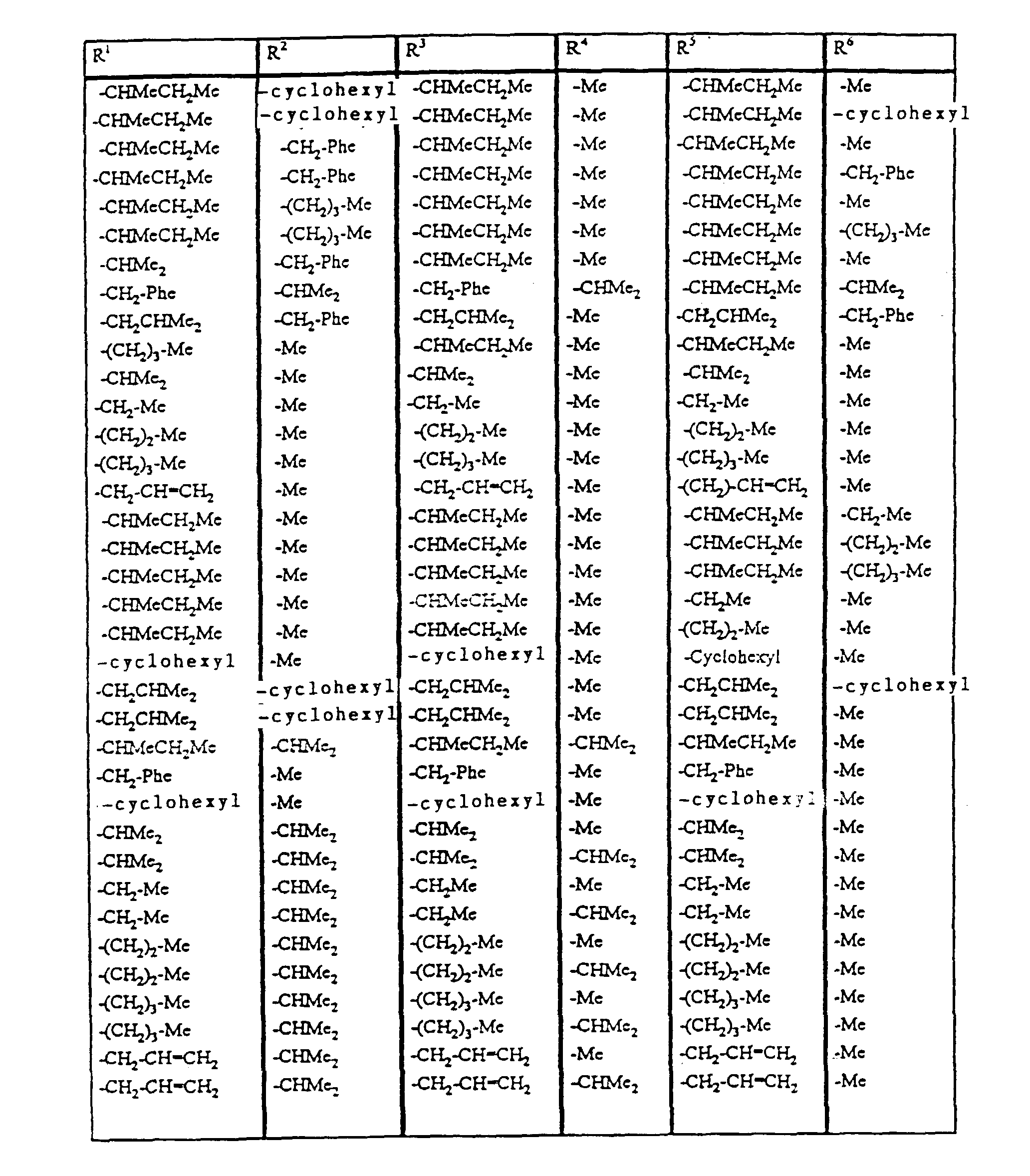

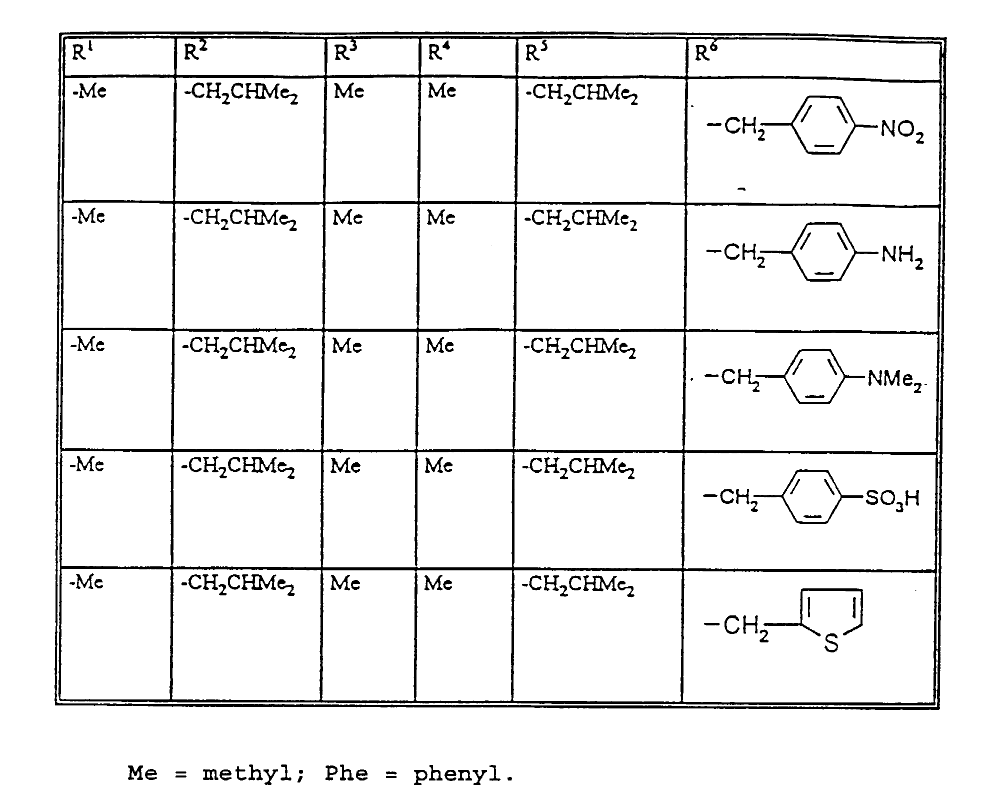

9. Process for the preparation of the new cyclic depsipeptides having 12 ring atoms, of the formula (I) according to Claim 6, 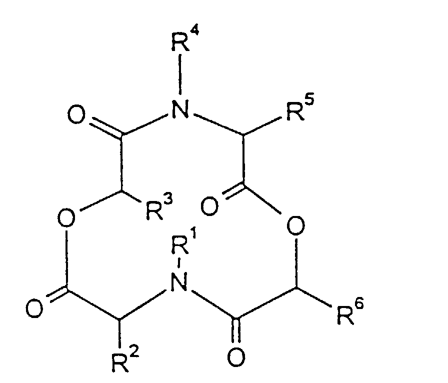 in which
the radicals R¹-R⁶ have the meanings given in Claim 6,
characterized in that open-chain tetradepsipeptides of the formula (II) 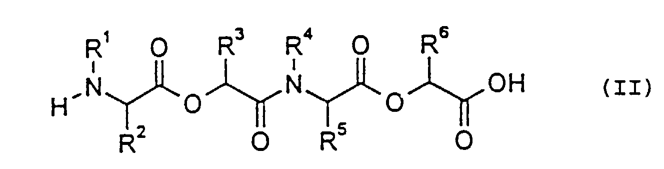 in which
R¹ to R⁶ have the abovementioned meanings,
are cyclized in the presence of a diluent and in the presence of a coupling reagent.

10. Cyclic depsipeptides having 12 ring atoms, of the formula (I) according to Claim 6, for use in endoparasiticides.

## Revendications

1. Utilisation de depsipeptides cycliques à noyau de 12 atomes, de formule générale (I) 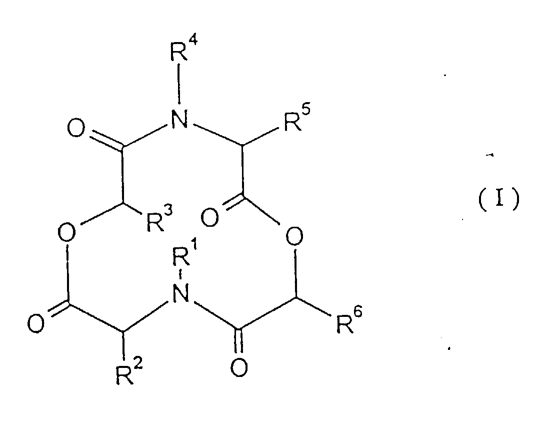 dans laquelle les restes ont les définitions suivantes :
R¹ et R⁴ représentent, indépendamment l'un de l'autre, de l'hydrogène, un groupe linéaire ou ramifié alkyle en C₁ à C₈, mono-pentahalogénalkyle en C₁ à C₄, hydroxyalkyle en C₁ à C₆, (alcanoyloxy en C₁ à C₄)-(alkyle en C₁ à C₆), (alkoxy en C₁ à C₄)-(alkyle en C₁ à C₆), phényl-(alkyloxy en C₁ à C₄)-(alkyle en C₁ à C₆), mercapto-alkyle en C₁ à C₆, (alkylthio en C₁ à C₄)-(alkyle en C₁ à C₆), (alkylsulfinyle en C₁ à C₄) - (alkyle en C₁ à C₆), (alkylsulfonyle en C₁ à C₄)-(alkyle en C₁ à C₆), carboxy-(alkyle en C₁ à C₆), (alkoxy en C₁ à C₄)-carbonyl-(alkyle en C₁ à C₆), Phényl(alkoxy en C₁ à C₄)-carbonyl-(alkyle en C₁ à C₆), carbamoyl-(alkyle en C₁ à C₆), aminoalkyle en C₁ à C₆, (alkyle en C₁ à C₄)-amino-(alkyle en C₁ à C₆), di-(alkyle en C₁ à C₄)-amino-(alkyle en C₁ à C₆), guanido-(alkyle en C₁ à C₆), (alkoxy en C₁ à C₄)-carbonylamino-(alkyle en C₁ à C₆), 9-fluorénylméthoxycarbonyl(Fmoc)amino(alkyle en C₁ à C₆), cycloalkyle en C₃ à C₇, (cycloalkyle en C₃ à C₇)-alkyle en C₁ à C₄, phényle, phényl-(alkyle en C₁ à C₄), ces groupes pouvant être substitués par des restes de la série halogéno, hydroxy, nitro, CN, alkoxy en C₁ à C₄, alkyle en C₁ à C₄ ;
R², R³ R⁵ et R⁶ représentent, indépendamment les uns des autres, de l'hydrogène, un groupe linéaire ou ramifié alkyle en C₁ à C₈, mono-pentahalogénalkyle en C₁ à C₄, hydroxyalkyle en C₁ à C₆, (alcanoyloxy en C₁ à C₄)-(alkyle en C₁ à C₆), (alkoxy en C₁ à C₄)-(alkyle en C₁ à C₆), Phényl-(alkyloxy en C₁ à C₄)-(alkyle en C₁ à C₆), mercapto-alkyle en C₁ à C₆, (alkylthio en C₁ à C₄)-(alkyle en C₁ à C₆), (alkylsulfonyle en C₁ à C₄)-(alkyle en C₁ à C₆), carboxy-(alkyle en C₁ à C₆), (alkoxy en C₁ à C₄)-carbonyl-(alkyle en C₁ à C₆), phényl-(alkoxy en C₁ à C₄)carbonyl(alkyle en C₁ à C₆), carbamoyl-(alkyle en C₁ à C₆), aminoalkyle en C₁ à C₆, (alkyle en C₁ à C₄)-amine-(alkyle en C₁ à C₆), di-(alkyle en C₁ à C₄)-amine-(alkyle en C₁ à C₆), alcényle en C₂ à C₈, cycloalkyle en C₃ à C₇, (cycloalkyle en C₃ à C₇)-(alkyle en C₁ à C₄), phényl-(alkyle en C₁ à C₄), thiénylméthyle, thiazolylméthyle, pyridylméthyle, ces groupes pouvant être substitués le cas échéant par des restes de la série halogéno, hydroxy, sulfonyle (SO₃H), CN, NO₂, amine, di(alkyle en C₁ à C₄)amino, alkoxy en C₁ à C₄, alkyle en C₁ à C₄,
ainsi que de leurs isomères optiques et de leurs racémates,
pour la préparation de compositions destinées à combattre des endoparasites en médecine humaine et en médecine vétérinaire.

2. Utilisation suivant la revendication 1, caractérisée en ce qu'elle met en jeu des composés de formule I dans laquelle les restes ont la définition suivante :
R¹ et R⁴ représentent, indépendamment l'un de l'autre, de l'hydrogène, un groupe linéaire ou ramifié alkyle en C₁ à C₈, mono-pentahalogénalkyle en C₁ à C₄, hydroxy-(alkyle en C₁ à C₆), (alcanoyloxy en C₁ à C₄)-(alkyle en C₁ à C₆), (alkoxy en C₁ à C₄)-(alkyle en C₁ à C₆), phényl-(alkyloxy en C₁ à C₄)-(alkyle en C₁ à C₆), (alkoxy en C₁ à C₄)-carbonylamino-(alkyle en C₁ à C₆), cycloalkyle en C₃ à C₇, (cycloalkyle en C₃ à C₇)-(alkyle en C₁ à C₄), phényl-(alkyle en C₁ à C₄), ces groupes pouvant être substitués le cas échéant par un ou plusieurs restes identiques ou différents indiqués dans la revendication 1 ;
R², R³, R⁵ et R6 R⁶ représentent indépendamment les uns des autres de l'hydrogène, un groupe linéaire ou ramifié alkyle en C₁ à C₈, mono-pentahalogénalkyle en C₁ à C₄, hydroxy-(alkyle en C₁ à C₆), phényl-(alkyloxy en C₁ à C₄)-(alkyle en C₁ à C₆), carboxy-(alkyle en C₁ à C₆), (alkoxy en C₁ à C₄)-carbonyl-(alkyle en C₁ à C₆), phényl-(alkoxy en C₁ à C₄)-carbonyl-(alkyle en C₁ à C₆), (alkylamino en C₁ à C₄)-(alkyle en C₁ à C₆), di-(alkyle en C₁ à C₄)amino(alkyle en C₁ à C₆), alcényle en C₂ à C₈, cycloalkyle en C₃ à C₇, (cycloalkyle en C₃ à C₇)-(alkyle en C₁ à C₄), phényle, phényl-(alkyle en C₁ à C₄), thiénylméthyle, ces groupes pouvant être substitués le cas échéant par un ou plusieurs restes identiques ou différents indiqués dans la revendication 1,
ainsi que leurs isomères optiques et leurs racémates.

3. Utilisation suivant la revendication 1, caractérisée en ce qu'elle met en jeu des composés de formule (I) dans laquelle les restes ont la définition suivante :
R¹ et R⁴ représentent, indépendamment l'un de l'autre, de l'hydrogène, un groupe alkyle en C₁ à C₈ linéaire ou ramifié, (cycloalkyle en C₃ à C₇)-(alkyle en C₁à C₄), phényl-(alkyle en C₁ à C₄),
R², R³ R⁵ et R⁶ représentent, indépendamment les uns des autres, de l'hydrogène, un groupe linéaire ou ramifié alkyle en C₁ à C₈, mono-pentahalogénalkyle en C₁ à C₄, alcényle en C₂ à C₈, (cycloalkyle en C₃ à C₇)-(alkyle en C₁ à C₄), phényl-(alkyle en C₁ à C₄), thiénylméthyle, ces groupes pouvant être substitués le cas échéant par un ou plusieurs restes identiques ou différents indiqués dans la revendication 1,
ainsi que leurs isomères optiques et leurs racémates.

4. Utilisation suivant la revendication 1 de composés de formule générale (I), dans laquelle
les restes R¹ à R⁶ ont la définition suivante :

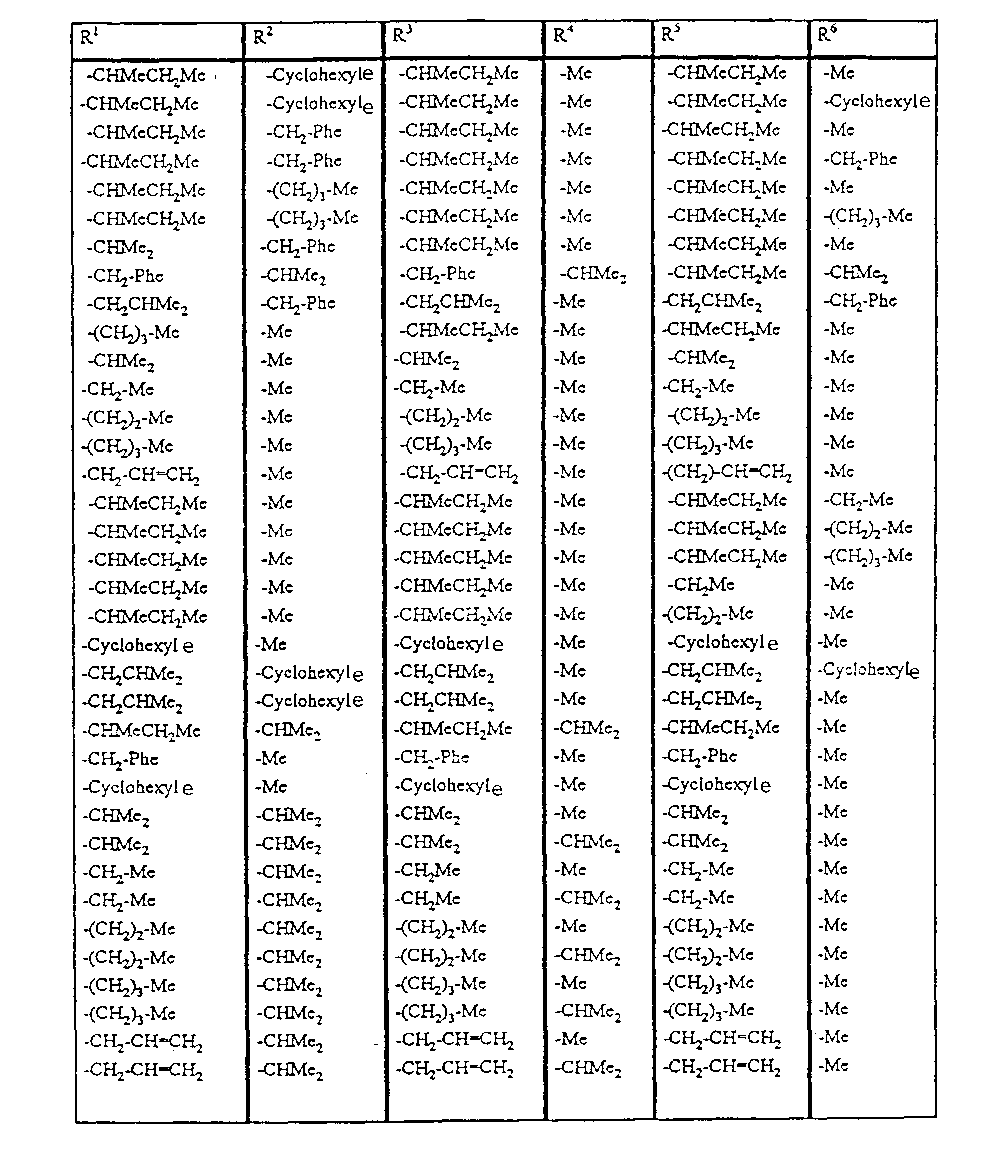

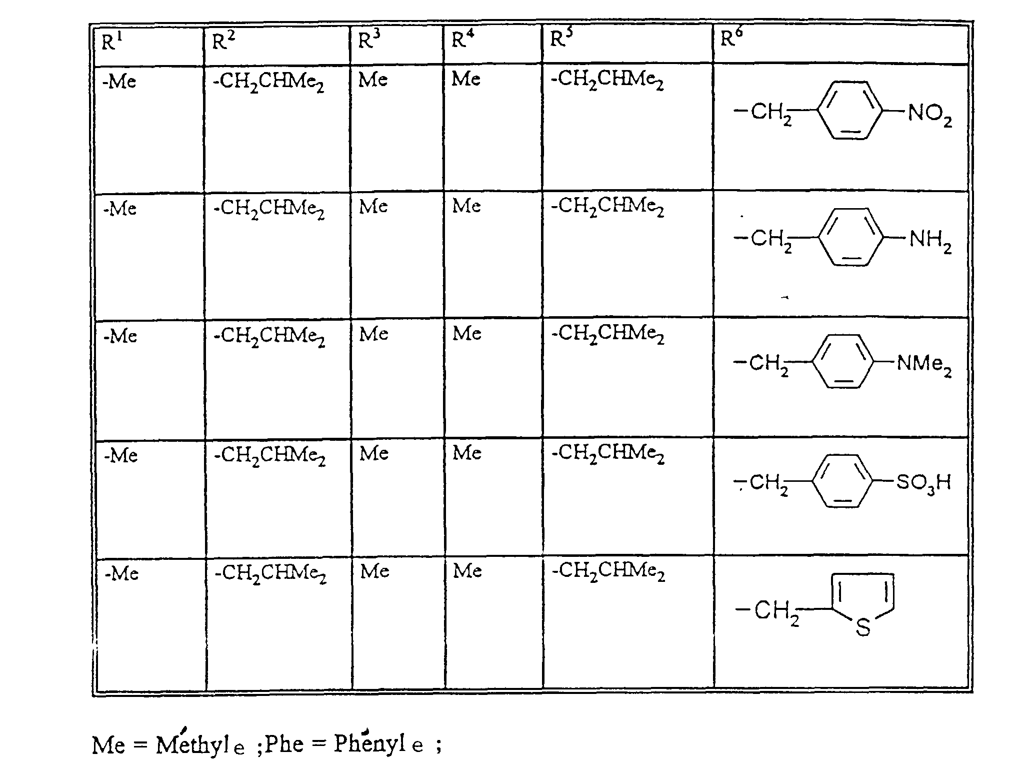

5. Compositions endoparasiticides, caractérisées par une teneur en au moins un depsipeptide cyclique à noyau de 12 atomes de formule (I) suivant les revendications 1 à 4.

6. Depsipeptides cycliques à noyau de 12 atomes de formule (I) 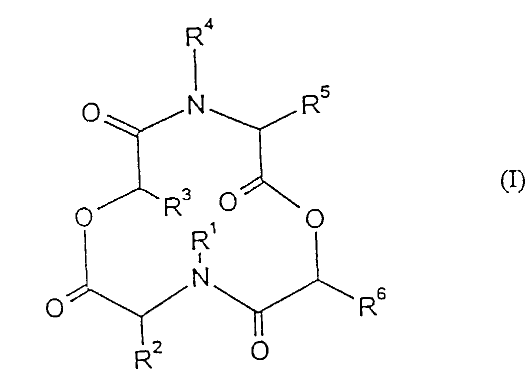 dans laquelle les restes ont la définition suivante
R¹ et R⁴ représentent, indépendamment l'un de l'autre, de l'hydrogène, un groupe linéaire ou ramifié alkyle en C₁ à C₈, mono-pentahalogénalkyle en C₁ à C₄, cycloalkyle en C₃ à C₆, phényle, phényl(alkyle en C₁ à C₄), ces groupes pouvant être substitués le cas échéant par des restes de la série halogéno, hydroxy, nitro, CN, alkoxy en C₁ à C₄, alkyle en C₁ à C₄,
R², R³, R⁵ représentent, indépendamment les uns des autres, de l'hydrogène, un groupe linéaire ou ramifié alkyle en C₁ à C₈, mono-pentahalogénalkyle en C₁ à C₄, hydroxyalkyle en C₁ à C₆, (alcanoyloxy en C₁ à C₄)-(alkyle en C₁ à C₆), (alkoxy en C₁ à C₄)-(alkyle en C₁ à C₆), mercapto-(alkyle en C₁ à C₆), (alkylthio en C₁ à C₄)-(alkyle en C₁ à C₆), (alkylsulfinyle en C₁ à C₄-(alkyle en C₁ à C₆), (alkylsulfonyle en C₁ à C₄)-(alkyle en C₁ à C₆), carboxy-(alkyle en C₁ à C₆), (alkoxy en C₁ à C₄)carbonyl-(alkyle en C₁ à C₆), phényl(alkoxy en C₁ à C₄)-carbonyl-(alkyle en C₁ à C₆), carbamoyl-(alkyle en C₁ à C₆), amino(alkyle en C₁ à C₆), (alkylamino en C₁ à C₄)-(alkyle en C₁ à C₆), di(alkyle en C₁ à C₄)amino(alkyle en C₁ à C₆), alcényle en C₂ à C₈, cycloalkyle en C₃ à C₇, (cycloalkyle en C₃ à C₇)-(alkyle en C₁ à C₄), phényle, phényl-(alkyle en C₁ à C₄), thiénylméthyle, thiazolylméthyle, pyridylméthyle, ces groupes pouvant être substitués le cas échéant par des restes de la série halogéno, hydroxy, sulfonyle iSO₃H), CN, NO₂, amino, di-(alkyle en C₁ à C₄)-amino, alkyle en C₁ à C₄ ;
R⁶ représente :
un groupe mono-pentahalogénalkyle en C₁ à C₄, hydroxy(alkyle en C₁ à C₆), (alcanoyloxy en C₁ C₄)-(alkyle en C₁ à C₆), (alkoxy en C₁ à C₄)-(alkyle en C₁ à C₆), mercapto-(alkyle en C₁ à C₆), (alkylthio en C₁ à C₄)-(alkyle en C₁ à C₆), (alkylsulfinyle en C₁ à C₄)-(alkyle en C₁ à C₆), (alkylsulfonyle en C₁ à C₄)-(alkyle en C₁ à C₆), carboxy-(alkyle en C₁ à C₆), (alkoxy en C₁ à C₄)-carbonyl-(alkyle en C₁ à C₆), phényl-(alkoxy en C₁ à C₄)-carbonyl-(alkyle en C₁ à C₆), carbamoyl-(alkyle en C₁ à C₆), aminoalkyle en C₁ à C₆, (alkylamino en C₁ à C₄)-(alkyle en C₁ à C₆), di-(alkyle en C₁ à C₄)-amino-(alkyle en C₁ à C₆), alcényle en C₂ à C₈, cycloalkyle en C₃ à C₇, (cycloalkyle en C₃ à C₇)-(alkyle en C₁ à C₄), phényle, phényl-(alkyle en C₁ à C₄), thiénylméthyle, thiazolylméthyle, pyridylméthyle, ces groupes pouvant être substitués le cas échéant par des restes de la série halogéno, hydroxy, sulfonyle (SO₃H), CN, NO₂, amino, di(alkyle en C₁ à C₄)amino, alkyle en C₁ à C₄ ;
ainsi que leurs isomères optiques et leurs racémates,
sous réserve qu'au cas où l'un des restes R¹ et R⁴ représente autre chose qu'un reste méthyle, le reste R⁶ puisse représenter, en plus des définitions indiquées, de l'hydrogène ou un groupe alkyle en C₁ à C₉ linéaire ou ramifié.

7. Depsipeptides cycliques suivant la revendication 6, dans la formule (I) desquels les restes ont la définition suivante :
R¹ et R⁴ représentent, indépendamment l'un de l'autre, de l'hydrogène, un groupe alkyle en C₁ à C₈ linéaire ou ramifié, un groupe phényl-(alkyle en C₁ à C₄), ces groupes pouvant éventuellement être substitués par un ou plusieurs des substituants indiqués dans la revendication 6 ;
R², R³, R⁵ représentent, indépendamment les uns des autres, de l'hydrogène, un groupe linéaire ou ramifié alkyle en C₁ à C₈, mono-pentahalogénalkyle en C₁ à C₄, alcényle en C₂ à C₈, (cycloalkyle en C₃ à C₇)-(alkyle en C₁ à C₄), phényl-(alkyle en C₁ à C₄), thiénylméthyle, ces groupes pouvant éventuellement être substitués par un ou plusieurs des substituants, identiques ou différents, indiqués dans la revendication 6 ;
R⁶ représente
un groupe mono-pentahalogénalkyle en C₁ à C₄, alcényle en C₂ à C₈, (cycloalkyle en C₃ à C₇)-(alkyle en C₁ à C₄), phényl-(alkyle en C₁ à C₄), thiénylméthyle, ces groupes pouvant être substitués le cas échéant par un ou plusieurs des restes indiqués dans la revendication 6, identiques ou différents,
ainsi que leurs isomères optiques et leurs racémates,
sous réserve qu'au cas où l'un des restes et R⁴ représente autre chose qu'un reste méthyle, le reste R⁶ puisse représenter, en plus des définitions indiquées, de l'hydrogène ou un reste alkyle en C₁ à C₉ linéaire ou ramifié.

8. Depsipeptides cycliques de formule (I) suivant la revendication 6, dans lesquels les restes R¹-R⁴ ont la définition suivante :

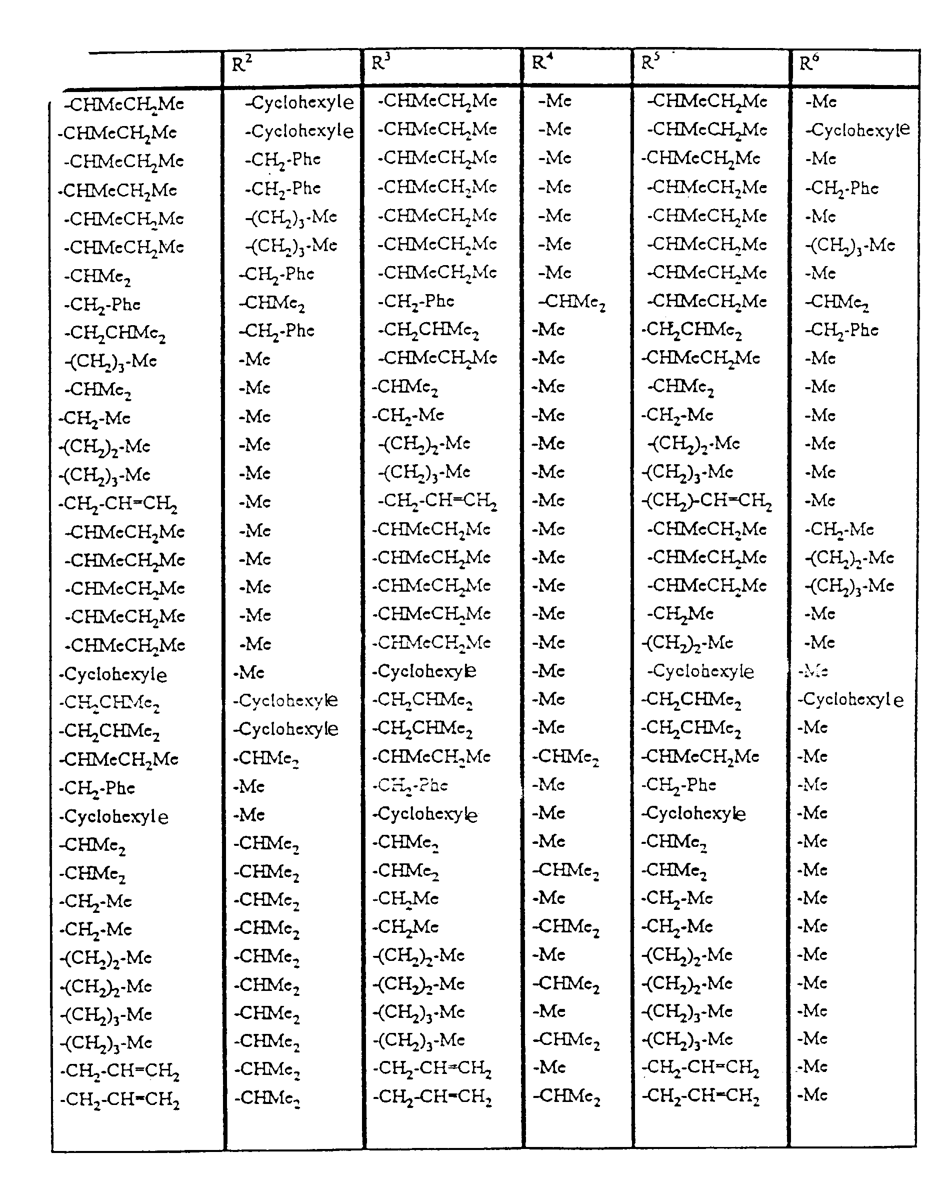

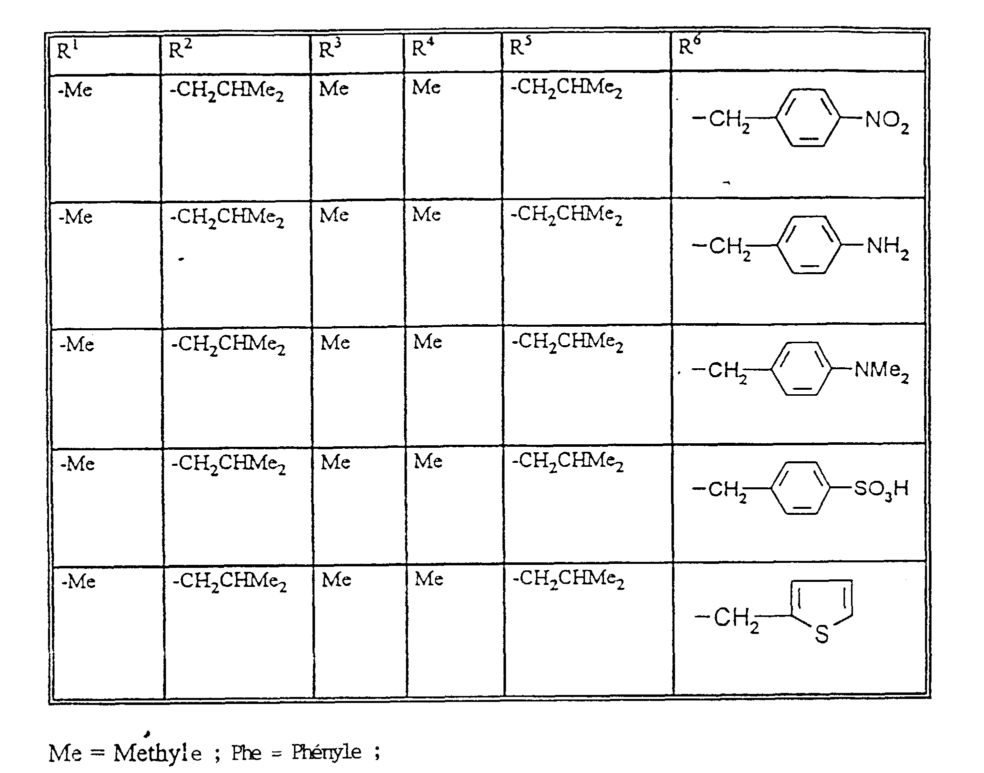

9. Procédé de production des depsipeptides cycliques nouveaux à noyau de 12 atomes de formule (I) suivant la revendication 6, 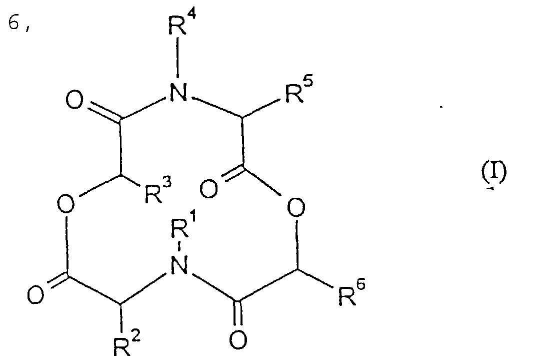 dans laquelle
les restes R¹ à R⁶ ont la définition indiquée dans la revendication 6,
caractérisé en ce qu'on cyclise des tétradepsipeptides à chaîne ouverte de formule (II) 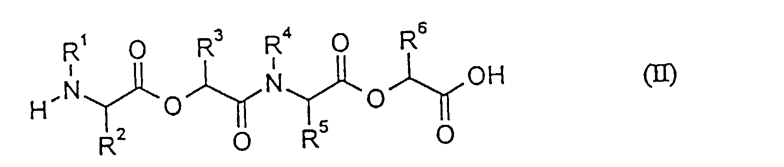 dans laquelle
R¹ à R⁶ ont la définition indiquée ci-dessus,
en présence d'un diluant et en présence d'un réactif de couplage.

10. Depsipeptides cycliques à noyau de 12 atomes de formule (I) suivant la revendication 6, destinés à être utilisés dans des compositions endoparasiticides.
